(19) Europäisches Patentamt
European Patent Office
Office européen des brevets

(11) Veröffentlichungsnummer: **0 248 348 A1**

# (12) EUROPÄISCHE PATENTANMELDUNG

(21) Anmeldenummer: **87107729.3**

(22) Anmeldetag: **27.05.87**

(51) Int. Cl.[4]: **A01N 43/54** , C07D 239/42

(30) Priorität: **31.05.86 DE 3618353**

(43) Veröffentlichungstag der Anmeldung: **09.12.87 Patentblatt 87/50**

(84) Benannte Vertragsstaaten: **AT BE CH DE ES FR GB GR IT LI NL SE**

(71) Anmelder: **HOECHST AKTIENGESELLSCHAFT**
**Postfach 80 03 20**
**D-6230 Frankfurt am Main 80(DE)**

(72) Erfinder: **Giencke, Wolfgang, Dr.**
**Am Steinberg 45**
**D-6238 Hofheim am Taunus(DE)**
Erfinder: **Mildenberger, Hilmar, Dr.**
**Fasanenstrasse 24**
**D-6233 Kelkheim (Taunus)(DE)**
Erfinder: **Heubach, Günther, Dr.**
**Luisenstrasse 15**
**D-6233 Kelkheim (Taunus)(DE)**
Erfinder: **Sachse, Burkhard, Dr.**
**An der Ziegelei 30**
**D-6233 Kelkheim (Taunus)(DE)**
Erfinder: **Fuss, Andreas, Dr.**
**Lindigstrasse 24**
**D-8757 Karlstein(DE)**
Erfinder: **Waltersdorfer, Anna, Dr.**
**Rauenthaler Weg 28**
**D-6000 Frankfurt am Main(DE)**
Erfinder: **Knauf, Werner, Dr.**
**Im Kirschgarten 24**
**D-6239 Eppstein/Taunus(DE)**
Erfinder: **Kern, Manfred, Dr.**
**Im Traminer Weg 8**
**D-6501 Lörzweiler(DE)**
Erfinder: **Bonin, Werner, Dr.**
**Im Schulzehnten 18**
**D-6233 Kelkheim (Taunus)(DE)**

(54) **Schädlingsbekämpfungsmittel auf der Basis von Aminopyrimidin-Derivaten sowie neue Amino-pyrimidin-Verbindungen.**

(57) Schädlingsbekämpfungsmittel, die eine Verbindung der Formel (I) enthalten,

EP 0 248 348 A1

Xerox Copy Centre

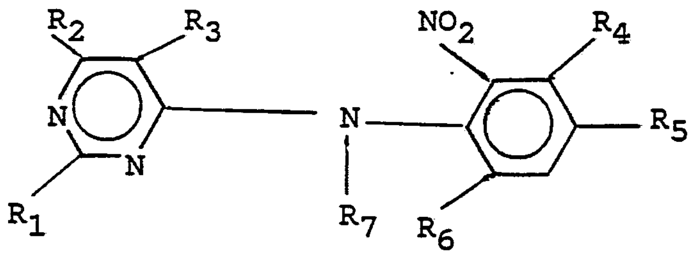

(I)

worin

$R_1$ und $R_2$ Wasserstoff; Halogen, Cyano; (Subst.)Alkyl; (Subst.)Cycloalkyl; (Halo)alkenyl; Cycloalkylenyl; eine Gruppe $N(R_8)(R_9)$; Alkylthio; (Halo)alkoxy; Alkylsulfonyl; (Subst.)Phenyl oder (Subst.)Phenoxy; $R_3$ Wasserstoff, Halogen; (Halo)alkyl; (Halo)alkenyl; Alkoxy; Alkylthio; Cyano; Nitro; (Subst.)Phenyl oder (Subst.) Phenoxy; $R_4$ Wasserstoff, Halogen, Alkylthio, (Subst.)Amino, Alkoxy oder (Subst.)Phenoxy; $R_5$, $R_6$ Nitro oder $CF_3$ bedeuten, eignen sich vorteilhaft zur Bekämpfung von tierischen Schädlingen im Pflanzenschutz. Verbindungen der Formel I, worin $R_1$ $CCl_3$ bedeuten, sind neu und stellen auch wertvolle Fungizide dar.

## Schädlingsbekämpfungsmittel auf der Basis von Aminopyrimidin-Derivaten sowie neue Aminopyrimidin-Verbindungen

N-(2-Nitrophenyl)-4-aminopyridin-Derivate zeigen fungizide und auch insektizider Wirkung (vgl. EP-A 31257).

Ferner ist bekannt, daß N-(2-Nitrophenyl)-4-aminopyrimidin-Derivate fungizide Eigenschaften besitzen (vgl. EP-A 139613).

Überraschenderweise wurde nun festgestellt, daß N-(2-Nitrophenyl)-4-aminopyrimidin-Derivate ausgezeichnete Insektizide sind, die sich vorteilhaft zum Einsatz im Pflanzenschutz eignen.

Gegenstand der vorliegenden Erfindung sind demnach Schädlingsbekämpfungsmittel, die eine Verbindung der allgemeinen Formel (I),

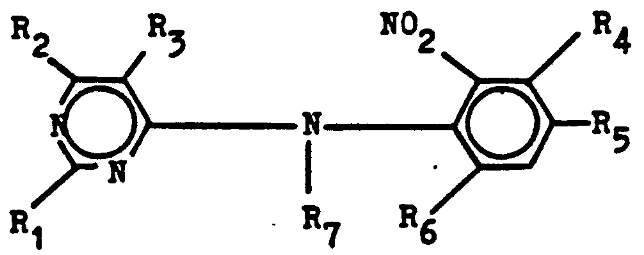

(I)

worin

$R_1$ und $R_2$ unabhängig voneinander Wasserstoff; Halogen; Cyano; $(C_1$-$C_8)$-Alkyl; $(C_1$-$C_8)$-Haloalkyl; $(C_1$-$C_8)$-Alkyl, das ein-oder zweifach durch Nitro, Cyano, $(C_1$-$C_4)$-Alkoxy, $(C_1$-$C_4)$-Alkylthio oder -$N(R_8)(R_9)$ substituiert ist; $(C_3$-$C_8)$-Cycloalkyl, das durch $(C_1$-$C_4)$-Alkyl substituiert sein kann; $(C_2$-$C_4)$-Alkenyl; $(C_2$-$C_4)$-Haloalkenyl; $(C_5$-$C_6)$-Cycloalkenyl; eine Gruppe $N(R_8)(R_9)$; $(C_1$-$C_8)$-Alkylthio; $(C_1$-$C_8)$-Alkoxy; $(C_1$-$C_8)$-Haloalkoxy; $(C_1$-$C_8)$-Alkylsulfonyl; Phenyl, das gegebenenfalls durch Halogen, Nitro, Cyano $(C_1$-$C_4)$-Alkyl, $(C_1$-$C_4)$-Alkoxy, $(C_1$-$C_4)$-Alkylthio oder $(C_1$-$C_4)$-Haloalkyl substituiert sein kann oder Phenoxy, das ein-bis einfach durch Halogen, $NO_2$, $(C_1$-$C_4)$-Alkyl, $(C_1$-$C_4)$-Haloalkyl oder $(C_1$-$C_4)$-Alkoxy substituiert sein kann;

$R_3$ Wasserstoff, Halogen; $(C_1$-$C_8)$-Alkyl; $(C_1$-$C_8)$-Haloalkyl; $(C_2$-$C_4)$-Alkenyl; $(C_2$-$C_4)$-Haloalkenyl; $(C_1$-$C_4)$-Alkoxy; $(C_1$-$C_4)$-Alkythio; Cyano; Nitro; Phenyl, das ein-bis dreifach durch Halogen, Nitro, Cyano, $(C_1$-$C_4)$-Alkyl, $(C_1$-$C_4)$-Alkoxy, $(C_1$-$C_4)$-Alkylthio oder $(C_1$-$C_4)$-Haloalkyl substituiert sein kann; oder Phenoxy, das ein-bis dreifach durch Halogen, $NO_2$, $(C_1$-$C_4)$-Alkyl, $(C_1$-$C_4)$-Haloalkyl oder $(C_1$-$C_4)$-Alkoxy substituiert sein kann,

$R_4$ Wasserstoff, Halogen, $(C_1$-$C_4)$-Alkoxy; $(C_1$-$C_4)$-Alkylthio, eine Gruppe $N(R_8)(R_9)$ oder Phenoxy, das ein-bis dreifach durch Halogen, Nitro, $(C_1$-$C_4)$-Alkyl, $(C_1$-$C_4)$-Haloalkyl oder $(C_1$-$C_4)$-Alkoxy substituiert sein kann;

$R_5$ und $R_6$ unabhängig voneinander Nitro oder $CF_3$;

$R_7$ Wasserstoff, eine Gruppe -$COR_{10}$ oder ein Kationäquivalent,

$R_8$ und $R_9$ unabhängig voneinander Wasserstoff, $(C_1$-$C_4)$-Alkyl oder $(C_3$-$C_7)$-Cycloalkyl und

$R_{10}$ Wasserstoff oder $(C_1$-$C_4)$-Alkyl bedeuten, enthalten.

Unter einem "Kationäquivalent" sind die in der Landwirtschaft einsetzbaren Kationen, insbesondere Alkali-oder Erdalkali-Ionen sowie gegebenenfalls ein-bis vierfach durch $(C_1$-$C_4)$-Alkyl oder $(C_1$-$C_4)$-Hydroxyalkyl substiutiertes Ammonium zu verstehen.

Der Begriff Halogen beinhaltet Fluor, Chlor, Brom und Jod.

Die Vorsilbe "Halo" in der Bezeichnung eines Substituenten bedeutet hier und im folgenden, daß dieser Substituent einfach oder mehrfach bei gleicher oder verschiedener Bedeutung auftreten kann. Die Vorsilbe Halo beinhaltet Fluor, Chlor, Brom und Jod, insbesondere Chlor und Brom.

Als Haloalkyl-Reste seien beispielsweise genannt; $CF_3$, $CHF_2$, $CH_2F$, $CCl_3$, $CHCl_2$, $CH_2Cl$, $CBr_3$, $CF_2Cl$, $CClF_2$, $CF_2CHF_2$, $CF_2CF_3$, $CF_2CHClF$, $CF_2CHCl_2$, $CCl_2$,$CCl_3$, n-$C_3F_7$, $CH(CF_3)_2$, $CF_2CHFCF_3$, n-$C_4F_9$, n-$C_5F_{11}$, n-$C_6F_{13}$, n-$C_7F_{15}$ und n-$C_8F_{17}$. Als Beispiele für $(C_2$-$C_4)$-Haloalkenyl seinen genannt: $CH_2=CF$-, $F_2C=CF$-und $Cl_2C=CCl$-.

Bevorzugte erfindungsgemäß einzusetzende Verbindungen der formel I sind solche, bei denen $R_1$, $R_2$ = $(C_1$-$C_8)$-Alkyl, $(C_1$-$C_8)$-Haloalkyl; $(C_1$-$C_8)$-Alkyl, das ein-oder zweifach durch Nitro, Cyano, $(C_1$-$C_4)$-Alkoxy, $(C_1$-$C_4)$-Alkylthio oder -$N(R_8)(R_9)$ substituiert ist, $(C_3$-$C_8)$-Cycloalkyl, das durch $(C_1$-$C_4)$-Alkyl substituiert sein kann; $(C_2$-$C_4)$-Alkenyl; $(C_2$-$C_4)$-Haloalkenyl; Phenyl, das gegebenenfalls durch Halogen, Nitro, Cyano, $(C_1$-$C_4)$-Alkyl, $(C_1$-$C_4)$-Alkoxy, $(C_1$-$C_4)$-Alkylthio oder $(C_1$-$C_4)$-Haloalkyl substituiert sein kann,

$R_3$ Wasserstoff, Halogen; $(C_1-C_8)$-Alkyl; $(C_1-C_8)$-Haloalkyl; Cyano oder Phenoxy, das ein-bis dreifach durch Halogen, $NO_2$, $(C_1-C_4)$-Alkyl, $(C_1-C_4)$-Haloalkyl oder $(C_1-C_4)$-Alkoxy substituiert sein kann;
$R_4$ Wasserstoff; Halogen, $(C_1-C_4)$-Alkoxy oder $(C_1-C_4)$-Alkylthio;
$R_5$ und $R_6$ unabhängig voneinander Nitro oder $CF_3$;
$R_7$ Wasserstoff, eine Gruppe $-COR_{10}$ oder ein Kationäquivalent;
$R_8$ und $R_9$ unabhängig von einander Wasserstoff; $(C_1-C_4)$-Alkyl oder $(C_3-C_7)$-Cycloalkyl und
$R_{10}$ Wasserstoff oder $(C_1-C_4)$-Alkyl bedeuten.

Insbesondere bevorzugt hiervon sind Verbindungen der Formel I, worin $R_1$ $(C_1-C_8)$-Haloalkyl, $R_2$ Wasserstoff, $R_3$ Halogen, $R_4$ Wasserstoff, $R_5$ und $R_6$ unabhängig voneinander $NO_2$ oder $CF_3$ und $R_7$ Wasserstoff bedeuten.

Verbindungen der Formel I, worin $R_1$ ausschließlich $CCl_3$ bedeutet und die Substituenten $R_2$, $R_3$, $R_4$, $R_5$, $R_6$, $R_7$, $R_8$, $R_9$ und $R_{10}$ die im Vorstehenden beschriebenen Bedeutungen haben, sind neu und werden ebenfalls von der Erfindung umfaßt. Diese Verbindungen zeigen ausgezeichnete fungizide Wirkungen.

Gegenstand der vorliegenden Erfindung sind auch Verfahren zur Herstellung der neuen Verbindungen der Formel I, worin $R_1$ ausschließlich $CCl_3$ bedeutet und die Substituenten $R_2$, $R_3$, $R_4$, $R_5$, $R_6$, $R_7$, $R_8$, $R_9$ und $R_{10}$ die im Vorstehenden beschriebenen Bedeutungen haben, dadurch gekennzeichnet, daß man eine Verbindungen der Formel II in Gegenwart einer Base mit einer Verbindung der Formel III umsetzt.

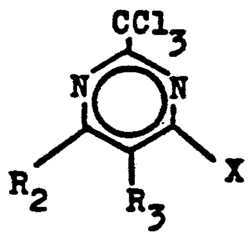 (II)

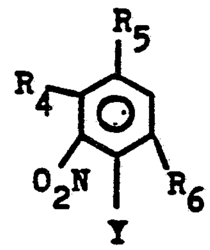 (III)

Die Substituenten $R_2$ und $R_3$ in Formel II, sowie die Substituenten $R_4$, $R_5$ und $R_6$ in Formel III haben die gleichen Bedeutungen wie in der Formel I. X und Y stehen für Halogen oder $NH_2$ wobei in dem Fall, in dem X für Halogen steht, Y $NH_2$ bedeutet und in dem Fall, in dem Y für Halogen steht, X $NH_2$ bedeutet.
Der Begriff Halogen repräsentiert Fluor, Chlor, Brom und Jod, insbesondere Cl und Br. Die oben genannten Vorzugsbereiche für $R_2$ bis $R_{10}$ gelten auch in diesem Zusammenhang. Insbesondere sind als Fungizide jedoch geeignet solche Verbindungen, bei denen $R_1 = CCl_3$, $R_2$ = Halogen, $(C_1-C_8)$-Alkyl, $(C_1-C_8)$-Haloalkyl; $R_3$ = H, Halogen, Cyano; $R_4$ = H oder Halogen; $R_5$, $R_6$ = Nitro oder $CF_3$ und $R_7$ = H bedeuten.

Die Umsetzung der Verbindung II mit III erfolgt vorzugsweise in inerten aprotischen Lösungsmitteln wie z.B. Actonitril, Dichlormethan, DMSO, Toluol, Xylol, Tetrahydrofuran, Dialkylether wie Diethylenglykoldimethylether, Dioxan oder Dioxan oder DMF bei Temperaturen zwischen -10 °C und der Siedetemperatur des Lösungsmittels. Als Basen eignen sich Carbonate und Hydrogencarbonate von Alkali-und Erdalkalimetallen, Alkalihydroxide, Alkalialkoholate wie K-tert.-butylat, tert. Amine, Pyridine und substituierte Pyridinbasen (z.B 4-Dimethylaminopyridin).

Auch eine zweites Äquivalent der Verbindungen der allgemeinen Formel II kann die Funktion der Base übernehmen. Als Basen kommen nur diejenigen Verbindungen der Formel II in Betracht, in denen X für $NH_2$ steht und $R_2$ und $R_3$ die im Vorstehenden genannten Bedeutungen haben.

Die Umsetzung erfolgt sowohl in aprotischen Lösungsmitteln wie z.B. Acetonitril, Dichlormethan, Toluol, Xylol, Tetrahydrofuran, Dioxan oder DMF als auch in protischen Lösungmitteln wie z.B. Methanol, Ethanol oder Isopropanol im Temperaturbereich von 0 °C bis zur Siedetemperatur des Lösungsmittels.

Die Verbindungen der Formel II lassen sich nach grundsätzlich bekannten Verfahren synthetisieren, vgl. z.B. US-PS 4 393 803 und J. Org. Chem. 26, 4504. Die Verbindungen der Formel III sind bekannt und ebenfalls nach Standardmethoden herstellbar.

Die erfindungsgemäßen Mittel eignen sich bei guter Pflanzenverträglichkeit und günstiger Warmblütertoxizität zur Bekämpfung von tierischen Schädlingen, insbesondere Insekten, Spinnentieren und Nematoden, ganz besonders bevorzugt zur Bekämpfung von Insekten, die in der Landwirtschaft, in Forsten, im Vorrats-und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören: Aus der Ordnung der Isopoda z.B. Oniscus asellus, Armadillidium vulgare, Porcellio scaber.

Aus der Ordnung der Diplopoda z.b. Blaniulus guttulatus.

Aus der Ordnung der Chilopoda z.B. Geophilus carpophagus, Scutigera spp.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blatella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Orndung der Isoptera z.B. Reticulitermes spp.,

Aus der Ordnung der Anoplura z.B. Phylloera vastatrix, Pemphigus spp., Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadratum, Cimex lectularius, Rhodinus prolixus, Triatoma spp.

Aus der Ordnung der Homoptera z.B. Aleurodes brassicae, Bemisis tabaci, Trialeurodes vaporariorum, Aphis gossypii, Brevicornyne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelus bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp., Bucculatrix thurberiella, Phyllocnistis citrella, Agrotis spp, Euxoa spp., Feltia spp., Earias insulana, Heliothis spp., Laphygma exigua, Mamestra brassicae, Panolis flamea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capus reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastics alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogster, Musca spp., Fannia spp., Calliphora erythrocephala, Lucilia spp., Chrysomyia spp., Cuterebra spp., Gastrophilus spp., Hypobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp.

Aus der Ordnung der Arachnida z.B. Scorpio maurus, Latrodectus mactans.

Auch können verschiedene Spinnmilbenarten, wie die Obstbaumspinnmilbe (Metatetranychus ulmi), die Citruspinnmilbe (Panonychus citri) und die Bohnenspinnmilbe (Tetranychus urticae), darunter auch Phosphorsäureester-resistene Stämme, gut bekämpft werden.

Auch gegen Ektoparasiten bei Tieren sind die Verbindungen der Formel I ausgezeichnet wirksam. Sie besitzen sowohl eine gute Wirkung gegen permanten und temporär parasitierende Insekten als auch gegen Milben und insbesondere gegen Zecken.

Zu den tierischen Ektoparasiten aus der Klasse der Insekten, gegen die die Verbindungen der Formel (I) wirksam sind, gehören Läuse (Anoplura), Flöhe (Ceratophyllidae), Haar-und Federlinge (Mallophaga), ferner Fliegen, wie Stechfliegen (Stomoxydidae) und Bremsen (Tabanidae), sowie solche Fliegen, deren Entwicklungsformen (Larven) als Schädlinge im Tierkörper parasitieren (Calliphoridae, Sarcophagidae, Gastrophilidae, Oestridae) und schließlich Lausfliegen (Hippoboscidae). Ektoparasiten aus der Ordnung der

Milben (Acari) sind die Räudemilben (Sarcoptidae), Vogelmilben (Dermanyssidae), Lederzecken (Argasidae) und schließlich die Schildzecken (Ixodidae), darunter besonders die einwirtigen Rinderzecken microplus und Boophilus decoloratus sowie die mehrwirtigen Arten der Gattungen Rhipicephalus, Amblyomma und Hyalomma.

Es wurde ferner überraschenderweise gefunden, daß bei der Kombination von Verbindungen der Formel I mit bekannten Insektiziden und Akariziden synergistische Wirkungen auftreten.

Gegenstand der Erfindung sind daher auch Schädlingsbekämpfungsmittel, die die Verbindungen der Formel I in Kombination mit einem Insektizid aus der Gruppe der Phosphorsäureester, Nitrophenol-Derivate, Diarylcarbinole, Carbamate, Formamidine, Carbonsäureester, Zinnverbindungen, Thiazolidinon Derivate oder Endosulfan enthalten. Darüber hinaus lassen sich die Verbindungen der Formel I auch mit anderen speziellen insektiziden Wirkstoffen, die im folgenden noch präzisiert werden, vorteilhaft kombinieren.

Von den erfindungsgemäß einzusetzenden Kombinationspartnern für die Verbindungen der Formel I seien genannt:

1. aus der Gruppe der Phosphorsäureester

Azinphos-ethyl, Azinphos-methyl, 1-(4-Chlorphenyl)-4-(O-ethyl, S-propyl)phosphoryloxypyrazol (TIA-230), Chlorpyrifos, Coumaphos, Demeton, Demeton-S-methyl, Diazinon, Dichlorvos, Dimethoat, Ethoprophos, Etrimfos, Fenitrothion, Fenthion, Heptenophos, Parathion, Parathion-methyl, Phosalon, Pirimiphos-ethyl, Pirimiphosmethyl, Profenofos, Prothiofos, Sulprofos, Triazophos, Trichlorphon.
Insbesonders bevorzugt sind Nicht-Thio-bzw. Dithiophosphorsäureester.

2. aus der Gruppe der Carbamate

Aldicarb, Bendiocarb, BPMC (2-(1-Methylpropyl)phenylmethylcarbamat), Butocarboxim, Butoxicarboxim, Carbaryl, Carbofuran, Carbosulfan, Cloethocarb, Methomyl, Isoprocarb, Oxamyl, Pirimicarb, Promecarb, Propoxur, Thiodicarb.

3. aus der Gruppe der Carbonsäureester

Allethrin, Alphametrin, Bioallethrin, Bioresmethrin, Cycloprothrin, Cyfluthrin, Cyhalothrin, Cypermethrin, Deltamethrin, 2,2-Dimethyl-3-(2-chlor-2-trifluormethylvinyl)cyclopropancarbonsäure-($\alpha$-cyano-3-phenyl-2-methylbenzyl)ester (FMC 54800), Fenpropathrin, Fenfluthrin, Fenvalerat, Flucythrinate, Flumethrin, Fluvalinate, Permethrin, Resmethrin, Tralomethrin.

4. aus der Gruppe der Formamidine

Amitraz, Chlordimeform

5. aus der Gruppe der Zinnverbindungen

Azocyclotin, Cyhexatin, Fenbutatinoxid

6. aus der Gruppe der Nitrophenol-Derivate

Binapacryl

7. aus der Gruppe der Diarylcarbinole

Dicofol

8. Andere geeignete Kombinationspartner für die Verbindungen der Formel I sind:

Bacillus thuringiensis, Bensultap, Bisclofentezin, Buprofecin, Cartrap, Cyromacin, Ethoproxyfen, Endosulfan, Fenoxycarb, Flubenzimin, Hexythiazox, 3-[2-(4-Ethoxyphenyl)-2-methyl-propoxymethyl ]-1,3-diphenylether (MTI-500), 5-[4-(4-Ethoxyphenyl)-4-methylpentyl ]-2-fluor-1,3-diphenylether (MTI-800), 3-(2-Chlorphenyl)-3-hydroxy-2-(2-phenyl-4-thiazolyl)-propennitril (SN 72129), Thiocyclam, Kernpolyeder-und Granuloseviren.

Die obengenannten Wirkstoffe, für die common names angegebenen wurden, sind in CH.R. Worthin, S.B. Walker, The Pe sticide Manual, 7th ed. Britisch Crop. Protection Council (1983) beschrieben.

Die übrigen Verbindungen werden in folgenden Veröffentlichungen beschrieben:

TIA-230 : IUPAC Kongreß-Bericht, Pesticide Chemistry 1982, Kyoto Japan.
FMC 54800 : H.J. Doel et al., FMC 54800, A New Acaricide-Insecticide, Symposium Gent (1984)
MTI 500, MTI 800 : Recent Advances in the Chemistry of Insect Control, Int., Symposium 25-27 Sept. 1984, Cambridge
SN 72129 : E.P. Pieters et al., Field Experiences with SN 72129, A New Selective Insecticide, 17th Int. Congress of Entomology 1984, Hamburg.

Die insektizide und akarizide Wirksamkeit der erfindungsgemäßen Wirkstoffkombinationen liegt teilweise deutlich höher als von den Wirkungen der Einzelkomponenten zu erwarten war. Durch Anwendung dieser Kombinationen können daher die Aufwandmengen der Einzelkomponenten reduziert werden. Ihre Anwendung bringt demzufolge ökonomische wie auch ökologische Vorteile.

Die erfindungsgemäßen Mittel eignen sich bei guter Pflanzenverträglichkeit und günstiger Warmblütertoxizität zur Bekämpfung der obengenannten Schädlinge.

Die Verbindungen der Formel I, insbesondere solche worin $R_1$ ausschließlich $CCl_3$ bedeutet und die Substituenten $R_2$, $R_3$, $R_4$, $R_5$, $R_6$, $R_7$, $R_8$, $R_9$ und $R_{10}$ die im Vorstehenden beschriebenen Bedeutungen haben, zeichnen sich auch durch eine hervorrangende fungizide Wirkung aus. Bereits in das pflanzliche Gewebe eingedrungene pilzliche Krankheitserreger lassen sich erfolgreich kurativ bekämpfen. Dies ist besonders wichtig und vorteilhaft bei solchen Pilzkrankheiten, die nach eingetretener Infektion mit den sonst üblichen Fungiziden nicht mehr wirksam bekämpft werden können. Das Wirkungsspektrum der beanspruchten Verbindungen erfaßt eine Vielzahl verschiedener wirtschaftlich wichtiger, phytophathogener Pilze, wie z.B. Piricularia oryzae, Pellicularia sasakii, verschiedene Rostarten, Venturia inaequalis, Cercospora-Arten, Plasmopara viticola, Pseudoperonospora cubensis und Phytophthora infestans. Besonders gut werden Benzimidazol-und Dicarboximidsensible und -resistente Botrytis cinerea-Stämme erfaßt, sowie BCM sensible und resistente Pseudocercosporella herpotrichoides Stämme.

Die Verbindungen der Formel I, insbesondere solche worin $R_1$ ausschließlich $CCl_3$ bedeutet und die Substituenten $R_2$, $R_3$, $R_4$, $R_5$, $R_6$, $R_7$, $R_8$, $R_9$ und $R_{10}$ die im Vorstehenden beschriebenen Bedeutungen haben, eignen sich auf für den Einsatz in technischen Bereichen, beispielsweise als Holzschutzmittel, als Konservierungsmittel in Anstrichfarben, in Kühlschmiermitteln für die Metallbearbeitung oder als Konservierungsmittel in Bohr-und Schneidölen.

Gegenstand der vorliegenden Erfindung sind daher auch fungizide Mittel, die Verbindungen der Formel I mit $R_1$ = $CCl_3$ enthalten.

Gegenstand der vorliegenden Erfindung sind auch Kombinationen der Verbindungen der Formel I mit einem weiteren Fungizid.

Als Fungizide, die erfindungsgemäß mit den Verbindungen der Formel I kombiniert werden können, sind folgende Wirkstoffe zu nennen.

Imazalil, Prochloraz, Fenapanil, SSF105, Triflumizol, PP969, Flutriafol, BAY-MEB 6401, Propiconazol, Etaconazol, Diclobutrazol, Bitertanol, Triadimefon, Triadimenol, Fluotrimazol, Tridemorph, Dodemorph, Fenpropimorph, Falimorph, S-32165, Chlobenzthiazone, Parinol, Buthiobat, Fenpropidin, Triforine, Fenarimol, Nuarimol, Triarimol, Ethirimol, Dimethirimol, Bupirimate, Rabenzazole, Tricyclazole, Ofurace, Furalaxyl, Benalazyl, Metalaxyl, Pencyuron, Oxadixyl, Cyprofuram, Dichlomezin, Probenazole, Fluobenzimine, Pyroxyfur, NK-483, PP-389, Pyroquilon, Hymexazole, Fenitropan, UHF-8227, Tolclofosmethyl, Ditalimfos, Edifenphos, Pyrazophos, Isoprothiolane, Cymoxanil, Dichloruanid, Captafol, Captan, Folpet, Tolylfluanid, Chlorothalonil, Etridiazol, Iprodione, Procymidon, Vinclozolin, Metomeclan, Myclozolin, Dichlozolinate, Fluorimide, Drazoxolon, Chinomethionate, Nitrothalisopropyl, Dithianon, Dinocap, Binapacryl, Fentinacetate, Fentinhydroxide, Carboxin, Oxycarboxin, Pyracarbolid, Methfuroxam, Fenfuram, Furmecyclox, Benodanil, Mebenil, Mepronil, Flutolanil, Fuberidazole, Thiabendazole, Carbendazim, Benomyl, Thiofanate, Thiofanate-methyl, CGO-94240F, IKF-1216, Mancozeb, Maneb, Zineb, Nabam, Thiram, Probineb, Prothiocarb, Propamocarb, Dodine, Guazatine, Dicloran, Quintozene, Chloroneb, Tecnazene, Biphenyl, Anilazine, 2-Phenylphenol, Kupferverbindungen wie Cu-oxychlorid, Oxine-Cu, Cu-oxide, Schwefel, Fosetylaluminium,
Natrium-dodecylbenzolsulfonat,
Natrium-dodecylsulfat,
Natrium-C13/C15-alkoholethersulfonat,
Natrium-cetostearylphosphatester,
Dioctyl-natriumsulfosuccinat,
Natrium-isopropylnaphthalinsulfonat,
Natrium-methylenbisnaphthalinsulfonat,
Cetyl-trimethyl-ammoniumchlorid,
Salze von langkettigen primären, sekündaren oder tertiären Aminen,
Alkyl propylenamine,
Lauryl-pyridiniumbromid,
Ethoxilierte quaternierte Fettamine,,
Alkyl-dimethyl-benzyl-ammoniumchlorid und
1-Hydroxyethyl-2-alkyl-imidazolin.

Die genannten Kombinationspartner stellen alle bekannte Wirkstoffe dar, die zum großen Teil in CH. R. Worthing, S.B. Walker, The Pesticide Manual, 7. Auflage (1983), British Crop Protection Council beschrieben sind. Verbindungen, für die Nummerncodes angegeben sind, besitzen folgende Strukturen:

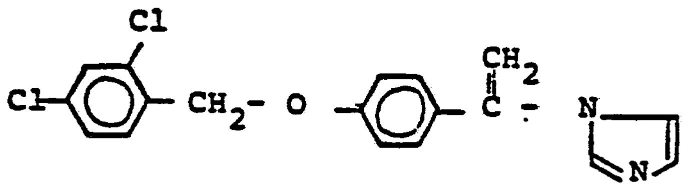

SS F 105

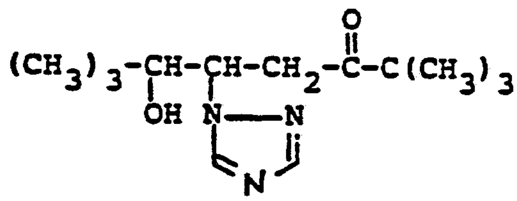

PP 969

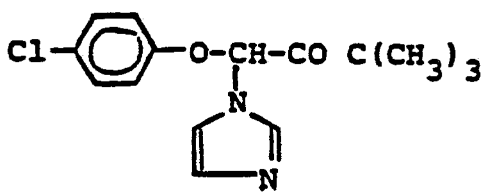

Bay-Meb 6401

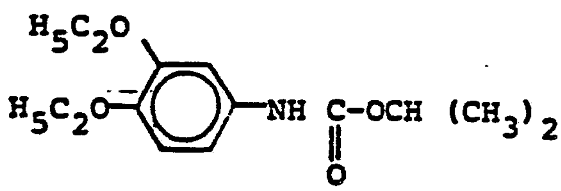

S - 32165

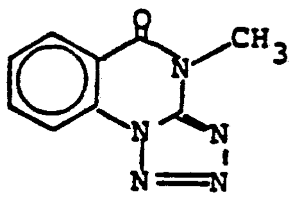

PP - 389

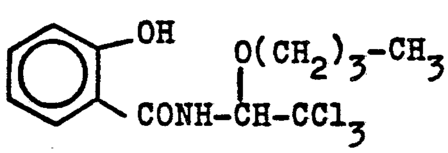

NK-483

Unerwarteterweise ist die fungizide Wirkung dieser Wirkstoffkombinationen in vielen Fällen höher, als die Summe der Wirkung der Einzelkomponenten, die nach der sogenannten Colby-Formel berechnet wird, vgl. S.R. Colby, Weeds 15, 20-22 (1967). Damit zeigen die Wirkstoffkombinationen synergistische Effekte. Die Gewichtsverhältnisse der Wirkstoffgruppen in den Wirkstoffkombinationen können in relativ großen Bereichen schwanken. Im allgemeinen entfallen auf 1 Gew.-Teil der Formel I 0,0005 bis 10 Gew.-Teile Wirkstoff, vorzugsweise 0,001 bis 2,5 Gew.-Teile des Kombinationspartners.

Die erfindungsgemäßen Wirkstoffkombinationen weisen eine starke biozide Wirkung aunf und können daher vorteilhaft zur Bekämpfung von Mikroorganismen, insbesondere im Pflanzenschutz, eingesetzt werden. Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz-und Saatgut und des Bodens.

Die erfindungsgemäßen Wirkstoffkombinationen haben ein sehr breites Wirkungsspektrum und können angewandt werden gegen parasitäre Pilze, die oberirdische Pflanzenteile befallen oder die Pflanzen vom Boden aus angreifen.

Die erfindungsgemäßen Wirkstoffkombinationen zeigen eine ausgezeichnete Wirkung als Saatgutbeizmittel gegen phytopathogene Pilze, wie z.B. Tilletia-, Urocystis-, Ustilago-, Septoria-, Typhula-, Rhynchosporium-, Helminthosporium-und Fusarium-Arten.

Auch als Bodenbekämpfungsmittel lassen sie sich zur Bekämpfung phytophathogener Pilze einsetzen, die Wurzelfäulen und Tracheomykosen verursachen wie z.B. Kranktheitserreger der Gattungen Pythium, Verticillium, Phialophora, Rhizoctonia, Fusarium und Thielaviopsis.

Bei direkter Applikation der Wirkstoffkombinationen auf die oberirdischen Pflanzenteile lassen sich eine Vielzahl wirstschaftlich wichtiger Krankheitserreger hervorrangend kontrollieren, wie z.B. echte Mehltaupilze (Erysiphe-, Uncinula-, Sphaerotheca-, Podosphaera-Arten, Leveillula taurica), Rostpilze, Ventura-Arten, Cercospora-Arten, Alternaria-Arten, Botrytis-Arten, Phytophthora-Arten, Peronospora-Arten, Pyricularia oryzae und Pellicularia sasakii.

Die erfindungsegemäßen Mittel können als Spritzpulver, emulgierbare Konzentrate, versprühbare Lösungen, Stäubemittel, Beizmittel, Dispersionen, Granulate oder Mikrogranlate in den üblichen Zubereitungen angewendet werden.

Spritzpulver sind in Wasser gleichmäßig dispergierbare Präparate, die neben dem Wirkstoff außer gegebenenfalls einem Verdünnungs-oder Inerstoff noch Netzmittel, z.B. polyoxethylierte Alkylphenole, polyoxethylierte Fettalkohole, Alkyl-oder Alkylphenylsulfonate und Dispergiermittel, z.B. ligninsulfonsaures Natrium, 2,2'-dinaphthylmethan-6,6'-disulfonsaures Natrium, dibutylnaphthalinsulfonsaures Natrium oder auch oleoylmethyltaurinsaures Natrium enthalten. Ihre Herstellung erfolgt in üblicher Weise z.B. durch Mahlen und Vermischen der Komponenten.

Emulgierbare Konzentrate können z.B. durch Auflösen des Wirkstoffes in einem inerten organischen Lösungsmittel, z.B. Butanol, Cyclohexanon, Dimethylformamid, Xylol oder auch höhersiedenden Aromaten oder Kohlenwasserstoffen unter Zusatz von einem oder mehreren Emulgatoren hergestellt werden. Bei flüssigen Wirkstoffen kann der Lösungsmittelanteil auch ganz oder teilweise entfallen. Als Emulgatoren können beispielsweise verwandt werden: Alkylarylsulfonsaure Calziumsalze wie Ca-dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanfettsäureester, Polyoxyethylensorbitanfettsäureester oder Polyoxethylensorbitester.

Stäubemittel kann man durch Vermahlen des Wirkstoffes mit fein verteilten festen Stoffen, z.B. Talkum, natürlichen Tonen wie Kaolin, Bentonit, Poryphillit oder Diatomeenerde erhalten.

Granulate könenn entweder durch Verdüsen des Wirkstoffes auf adsorptionsfähiges, granuliertes Inertmaterial hergestellt werden oder durch Aufbringen von Wirkstoffkonzentrationen mittels Bindemitteln, z.B. Polyvinylalkohol, polyacrylsaurem Natrium oder auch Mineralölen auf die Oberfläche von Trägerstoffen wie Sand, Kaolinite, oder von granuliertem Inertmaterial. Auch können geeignete Wirkstoffe in der für die Herstellung von Düngemittelgranulaten üblichen Weise - gewünschtenfalls in Mischung mit Düngemitteln - formuliert werden.

In Spritzpulvern beträgt die Wirkstoffkonzentration z.B. etwa 10 bis 90 Gew.-%; der Rest zu 100 Gew.-% besteht aus üblichen Formulierungsbestandteilen. Bei emulgierbaren Konzentraten kann die Wirkungstoffkonzentration etwa 10 bis 80 Gew.-% betragen.

Staubförmige Formulierungen enthalten meistens 5 bis 20 Gew.-% an Wirkstoff, versprühbare Lösungen etwa 2 bis 20 Gew.-%. Bei Granulaten hängt der Wirkstoffgehalt zum Teil davon ab, ob die wirksame Verbindung flüssig oder fest vorliegt und welche Granulierhilfsmitel, Füllstoffe usw. verwendet werden.

Daneben enthalten die genannten Wirkstofformulierungen gegebenenfalls die jeweils üblichen Haft-, Netz-, Dispergier-, Emulgier-, Penetrations-, Lösungsmittel, Füll-oder Trägerstoffe.

Zur Anwendung werden die in handelsüblicher Form vorliegenden Konzentrate gegebenenfalls in üblicher Weise verdünnt, z.B. bei Spritzpulvern, emulgierbaren Konzentraten, Dispersionen und teilweise auch bei Mikrogranulaten mittels Wasser. Staubförmige und granulierte Zubereitungen sowie versprühbare Lösungen werden vor der Anwendung üblicherweise nicht mehr mit weiteren inerten Stoffen verdünnt.

Auch Mischungen oder Mischformulierungen mit anderen Wirkstoffen, wie z.B. Insektiziden, Akariziden, Herbiziden, Düngemitteln und Wachstumsregulatoren sind gegebenenfalls möglich.

Die benötigten Aufwandmengen der Wirkstoffe der Formel I können je nach Indikation innerhalb weiter Grenzen schwanken und varrieren auch in Abhängigkeit von äußeren Bedingungen wie Bodenverhältnissen und Klimabedingungen. Im allgemeinen liegen sie jedoch zwischen 0,005 - 10kg Wirkstoff/ha.

Die Erfindung wird durch nachstehende Beispiele erläutert.

A. Formulierungsbeispiele

Beispiel A:

Ein Stäubemittel wird erhalten, indem man
10 Gewichtsteile Wirkstoff
und 90 Gewichtsteile Talkum
als Interstoff mischt und in einer Schlagmühle zerkleinert.

Beispiel B:

Ein in Wasser leicht dispergierbares, benetzbares Pulver wird erhalten, indem man
25 Gewichtsteile Wirkstoff
64 Gewichtsteile kaolinhaltigen Quarz als Inerstoff
10 Gewichtsteile ligninsulfonsäures Kalium
und 1 Gewichtsteil olioylmethyltaurinsaures Natrium als Netz-und Dispergiermittel
mischt und in einer Stiftmühle mahlt.

Beispiel C:

Ein in Wasser leicht dispergierbares Dispersionskonzentrat wird erhalten, indem man
20 Gewichtsteile Wirkstoff mit
6 Gewichtsteile Alkylphenolpolyglykolether (Trition X 207)
3 Gewichtsteilen Isotridecanolpolyglykoläther (8 AeO)
und 71 Gewichtsteilen paraffinischem Mineralöl (Siedebereich z.B. ca. 255 bis über 377 °C)
mischt und in einer Reibkugelmühle auf eine Feinheit von unter 5 Mikron vermahlt.

Beispiel D:

Ein emulgierbares Konzentrat wird erhalten aus
15 Gewichtsteilen Wirkstoff
75 Gewichtsteilen Cyclohexanon als Lösungsmittel
und 10 Gewichtsteilen oxäthyliertes Nonylphenol (10 AeO) als Emulgator.

Beispiel E:

Ein Stäubemittel wird erhalten, in dem man
20 % Wirkstoff
20 % Kombinationspartner
10 % ®Dispersogen A (Kondensationsprodukt von Naphthalinsulfonsäure und Formaldehyd)
15 % Zellpech
5 % ®Leonil DB (Na-Salz einer Alkylnaphthalinsulfonsäure)
2 % ®Hostapon T (Na-Salz von Oleylmethyltaurid)
5 % ® Mowiol 3-83 (teilverseiftes Polyvinylacetat)
und 23 % ®Sipernat 225 (synthet. $SiO_2$)
mischt und in einer Schlagkreuzmühle vermahlt.

Beispiel F:

Ein emulgierbares Konzentrat wird erhalten aus 10 % Wirkstoff, 10 % Kombinationspartner, 10 % ®Emulsogen EC 400, 50 % Phenylsulfonat-Ca, 30 % Xylol und 35 % Isophoron.

B. Chemische Beispiele

Beispiele für Verbindungen der Formel I sind in den Tabellen 1 bis 11 beschrieben.

Beispiele für die neuen Verbindungen der Formel I, worin $R_1$ ausschließlich $CCl_3$ bedeutet und die Substituenten $R_2$, $R_3$, $R_4$, $R_5$, $R_6$, $R_7$, $R_8$, $R_9$ und $R_{10}$ die im Vorstehenden beschriebenen Bedeutungen haben, sind in der Tabelle 11 aufgelistet.

Tabelle 1

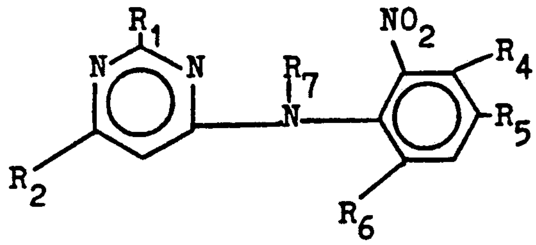

| Nr. | $R_1$ | $R_2$ | $R_4$ | $R_5$ | $R_6$ | $R_7$ | physik. Konstanten [°C] |
|---|---|---|---|---|---|---|---|
| 1.1 | H | H | H | $CF_3$ | $NO_2$ | H | Smp. 156 |
| 1.2 | H | Cl | Cl | $CF_3$ | $NO_2$ | H | Smp. 97-100 |
| 1.3 | $CH_3$ | H | Cl | $CF_3$ | $NO_2$ | H | |
| 1.4 | 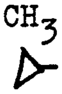 | H | H | $CF_3$ | $NO_2$ | H | |
| 1.5 | $CF_3$ | H | H | $CF_3$ | $NO_2$ | H | Harz |
| 1.6 | $C_2F_5$ | H | H | $CF_3$ | $NO_2$ | H | |
| 1.7 | $CF_2CHF_2$ | H | H | $CF_3$ | $NO_2$ | H | Smp. 206-208 |
| 1.8 | $C_3F_7$ | H | H | $CF_3$ | $NO_2$ | H | |
| 1.9 | H | $OCH_2CF_3$ | Cl | $CF_3$ | $NO_2$ | H | Smp. 98-100 |
| 1.10 | $CF_3$ | H | Cl | $CF_3$ | $NO_2$ | H | Sirup |
| 1.11 | $C_2F_5$ | H | Cl | $CF_3$ | $NO_2$ | H | |
| 1.12 | $CF_2CHF_2$ | H | Cl | $CF_3$ | $NO_2$ | H | |
| 1.13 | $CH_3$ | H | H | $CF_3$ | $NO_2$ | H | Smp. 109-111 |
| 1.14 | H | $SCH_3$ | Cl | $CF_3$ | $NO_2$ | H | Smp. 135-137 |
| 1.15 | H | $OCH_3$ | Cl | $CF_3$ | $NO_2$ | H | Smp. 128-129 |
| 1.16 | n-$C_3H_7$ | H | H | $CF_3$ | $NO_2$ | H | |
| 1.17 | n-$C_3H_7$ | H | Cl | $CF_3$ | $NO_2$ | H | |
| 1.18 | $C_6H_5$ | H | H | $CF_3$ | $NO_2$ | H | |
| 1.19 | $CF_3$ | H | Cl | $CF_3$ | $NO_2$ | H | Harz |
| 1.20 | $CF_3$ | H | H | $NO_2$ | $CF_3$ | H | Harz |
| 1.21 | $C_2H_5$ | H | Cl | $CF_3$ | $NO_2$ | H | |
| 1.22 | C≡N | H | H | $CF_3$ | $NO_2$ | H | |

Fortsetzung der Tabelle 1

| Nr. | $R_1$ | $R_2$ | $R_4$ | $R_5$ | $R_6$ | $R_7$ | physik. konstanten [°C] |
|---|---|---|---|---|---|---|---|
| 1.23 | C≡N | H | Cl | $CF_3$ | $NO_2$ | H | |
| 1.24 | H | H | H | $NO_2$ | $CF_3$ | H | semikristallin |
| 1.25 | $CF_2CHF_2$ | $CH_3$ | H | $CF_3$ | $NO_2$ | H | |
| 1.26 | $CF_2CHF_2$ | $CH_3$ | Cl | $CF_3$ | $NO_2$ | H | |
| 1.27 | $CF_3$ | H | H | $CF_3$ | $NO_2$ | K | |
| 1.28 | $C_2F_5$ | H | H | $CF_3$ | $NO_2$ | Na | |
| 1.29 | $CF_2CHF_2$ | H | H | $CF_3$ | $NO_2$ | K | |
| 1.30 | $C_3F_7$ | H | H | $CF_3$ | $NO_2$ | K | |
| 1.31 | $CF_2CHF_2$ | Cl | H | $NO_2$ | $CF_3$ | H | Smp. 108-109 |
| 1.32 | $CF_2CHF_2$ | Cl | H | $CF_3$ | $NO_2$ | H | Smp. 207-208 |

Tabelle 2

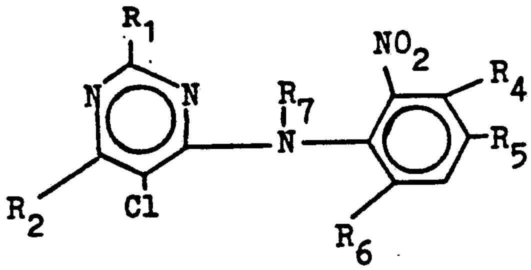

| Nr. | $R_1$ | $R_2$ | $R_4$ | $R_5$ | $R_6$ | $R_7$ | physik. Konstanten [°C] |
|---|---|---|---|---|---|---|---|
| 2.1 | $CH_3$ | H | H | $CF_3$ | $NO_2$ | H | Smp. 115-116 |
| 2.2 | $C_2H_5$ | H | H | $CF_3$ | $NO_2$ | H | |
| 2.3 | n-$C_3H_7$ | H | H | $CF_3$ | $NO_2$ | H | |
| 2.4 | i-$C_3H_7$ | H | H | $CF_3$ | $NO_2$ | H | |
| 2.5 | 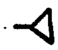 | H | H | $CF_3$ | $NO_2$ | H | |
| 2.6 | $CH=CH_2$ | H | H | $CF_3$ | $NO_2$ | H | |
| 2.7 | $CH_2Cl$ | H | H | $CF_3$ | $NO_2$ | H | |
| 2.8 | $CHCl_2$ | H | H | $CF_3$ | $NO_2$ | H | |
| 2.9 | SEt | H | H | $CF_3$ | $NO_2$ | H | |
| 2.10 | $CCl_2CHCl_2$ | H | H | $CF_3$ | $NO_2$ | H | |
| 2.11 | $CCl_2CCl_3$ | H | H | $CF_3$ | $NO_2$ | H | |
| 2.12 | $CF_3$ | H | H | $CF_3$ | $NO_2$ | H | Smp. 129-132 |
| 2.13 | $CF_2CHF_2$ | H | H | $CF_3$ | $NO_2$ | H | Smp. 139-140 |
| 2.14 | $CF_2CF_3$ | H | H | $CF_3$ | $NO_2$ | H | Smp. 133-135 |
| 2.15 | $C_3F_7$ | H | H | $CF_3$ | $NO_2$ | H | Smp. 91-93 |
| 2.16 | $CH(CF_3)_2$ | H | H | $CF_3$ | $NO_2$ | H | |
| 2.17 | $C_7F_{15}$ | H | H | $CF_3$ | $NO_2$ | H | Sirup |
| 2.18 | $CF_2Cl$ | H | H | $CF_3$ | $NO_2$ | H | Smp. 165-167 |
| 2.19 | $CFCl_2$ | H | H | $CF_3$ | $NO_2$ | H | |
| 2.20 | $CF_2CHCl_2$ | H | H | $CF_3$ | $NO_2$ | H | |
| 2.21 | $CF_2CHClF$ | H | H | $CF_3$ | $NO_2$ | H | Smp. 103-105 |
| 2.22 | $CF=CH_2$ | H | H | $CF_3$ | $NO_2$ | H | |
| 2.23 | $CF=CF_2$ | H | H | $CF_3$ | $NO_2$ | H | |
| 2.24 | $CCl=CCl_2$ | H | H | $CF_3$ | $NO_2$ | H | |

1. Fortsetzung der Tabelle 2

| Nr. | $R_1$ | $R_2$ | $R_4$ | $R_5$ | $R_6$ | $R_7$ | physik. Konstanten [°C] |
|---|---|---|---|---|---|---|---|
| 2.25 | OEt | H | Cl | $CF_3$ | $NO_2$ | H | Smp. 149-151 |
| 2.26 | $OCH_2CF_3$ | H | Cl | $CF_3$ | $NO_2$ | H | Smp. 146-147 |
| 2.27 | $n-C_3H_7$ | H | Cl | $CF_3$ | $NO_2$ | H | |
| 2.28 | $i-C_3H_7$ | H | Cl | $CF_3$ | $NO_2$ | H | |
| 2.29 | 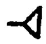 | H | Cl | $CF_3$ | $NO_2$ | H | |
| 2.30 | $CH=CH_2$ | H | Cl | $CF_3$ | $NO_2$ | H | |
| 2.31 | $CH_2Cl$ | H | Cl | $CF_3$ | $NO_2$ | H | |
| 2.32 | $CHCl_2$ | H | Cl | $CF_3$ | $NO_2$ | H | |
| 2.33 | $SCH(CH_3)_2$ | H | Cl | $CF_3$ | $NO_2$ | Na | |
| 2.34 | $CCl_2CHCl_2$ | H | Cl | $CF_3$ | $NO_2$ | H | |
| 2.35 | $CCl_2CCl_3$ | H | Cl | $CF_3$ | $NO_2$ | H | |
| 2.36 | $CF_3$ | H | Cl | $CF_3$ | $NO_2$ | H | Smp. 137-139 |
| 2.37 | $CF_2CHF_2$ | H | Cl | $CF_3$ | $NO_2$ | H | |
| 2.38 | $CF_2CF_3$ | H | Cl | $CF_3$ | $NO_2$ | H | |
| 2.39 | $C_3F_7$ | H | Cl | $CF_3$ | $NO_2$ | H | |
| 2.40 | $CH(CF_3)_2$ | H | Cl | $CF_3$ | $NO_2$ | H | |
| 2.41 | $C_7F_{15}$ | H | Cl | $CF_3$ | $NO_2$ | H | |
| 2.42 | $CF_2Cl$ | H | Cl | $CF_3$ | $NO_2$ | H | Smp. 147-148 |
| 2.43 | $CFCl_2$ | H | Cl | $CF_3$ | $NO_2$ | H | |
| 2.44 | $CF_2CHCl_2$ | H | Cl | $CF_3$ | $NO_2$ | H | |
| 2.45 | $CF_2CHClF$ | H | Cl | $CF_3$ | $NO_2$ | H | |
| 2.46 | $CF=CH_2$ | H | Cl | $CF_3$ | $NO_2$ | H | |
| 2.47 | $CF=CF_2$ | H | Cl | $CF_3$ | $NO_2$ | H | |
| 2.48 | $CCl=CCl_2$ | H | Cl | $CF_3$ | $NO_2$ | H | |
| 2.49 | $CF_3$ | H | H | $NO_2$ | $CF_3$ | H | Smp. 115-116 |
| 2.50 | $CF_3$ | Cl | H | $NO_2$ | $CF_3$ | H | |
| 2.51 | $CF_2CHF_2$ | H | H | $NO_2$ | $CF_3$ | H | Smp. 106-107 |
| 2.52 | $CF_2CF_3$ | H | Cl | $NO_2$ | $CF_3$ | H | |
| 2.53 | Cl | H | Cl | $CF_3$ | $NO_2$ | H | Smp. 176-177 |
| 2.54 | $OCH_3$ | H | Cl | $CF_3$ | $NO_2$ | H | Smp. 157-158 |
| 2.55 | $CH_3$ | H | Cl | $CF_3$ | $NO_2$ | H | |
| 2.56 | $C_2H_5$ | H | Cl | $CF_3$ | $NO_2$ | H | |

2. Fortsetzung der Tabelle 2

| Nr. | $R_1$ | $R_2$ | $R_4$ | $R_5$ | $R_6$ | $R_7$ | physik. Konstanten [°C] |
|---|---|---|---|---|---|---|---|
| 2.57 | H | H | Cl | $CF_3$ | $NO_2$ | H | |
| 2.58 | H | H | H | $CF_3$ | $NO_2$ | H | |
| 2.59 | $CF_2CHF_2$ | $CH_3$ | H | $CF_3$ | $NO_2$ | H | |
| 2.60 | $CF_2CHF_2$ | $CH_3$ | Cl | $CF_3$ | $NO_2$ | H | |
| 2.61 | $CF_2CF_3$ | $CH_3$ | H | $CF_3$ | $NO_2$ | H | |
| 2.62 | $CF_2CF_3$ | $CH_3$ | Cl | $CF_3$ | $NO_2$ | H | |
| 2.63 | $C\equiv N$ | $CH_3$ | Cl | $CF_3$ | $NO_2$ | H | |
| 2.64 | $C\equiv N$ | $CH_3$ | H | $CF_3$ | $NO_2$ | H | |
| 2.65 | $C\equiv N$ | H | H | $CF_3$ | $NO_2$ | H | |
| 2.66 | $C\equiv N$ | H | Cl | $CF_3$ | $NO_2$ | H | |
| 2.67 | 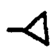 | H | Cl | $CF_3$ | $NO_2$ | K | |
| 2.68 | $CHCl_2$ | H | H | $CF_3$ | $NO_2$ | K | |
| 2.69 | $CF_3$ | H | H | $CF_3$ | $NO_2$ | K | |
| 2.70 | $CF_3$ | H | Cl | $CF_3$ | $NO_2$ | Na | |
| 2.71 | $CF_2CHF_2$ | H | Cl | $CF_3$ | $NO_2$ | K | Smp. 272-274 |
| 2.72 | $CF_2CF_3$ | H | Cl | $CF_3$ | $NO_2$ | K | Smp. 276-279 |
| 2.73 | $CFCl_2$ | H | Cl | $CF_3$ | $NO_2$ | K | |
| 2.74 | $CClF_2$ | H | H | $CF_3$ | $NO_2$ | K | Smp. 271-273 |
| 2.75 | $CClF_2$ | H | Cl | $CF_3$ | $NO_2$ | K | Smp. 273-276 |
| 2.76 | $CF_2CHF_2$ | H | H | $CF_3$ | $NO_2$ | K | Smp. 267-270 |
| 2.77 | $CF_2CF_3$ | H | H | $CF_3$ | $NO_2$ | K | Smp. 273-275 |
| 2.78 | $CH_3$ | H | H | $CF_3$ | $NO_2$ | Na | |
| 2.79 | $CH_3$ | H | H | $CF_3$ | $NO_2$ | K | |
| 2.80 | $C_3F_7$ | H | H | $CF_3$ | $NO_2$ | K | |
| 2.81 | $CClF_2$ | H | H | $CF_3$ | $NO_2$ | K | |
| 2.82 | $C_7F_{15}$ | Cl | Cl | $CF_3$ | $NO_2$ | K | |
| 2.83 | $C_7F_{15}$ | H | H | $CF_3$ | $NO_2$ | K | |
| 2.84 | $C_7F_{15}$ | H | H | $CF_3$ | $NO_2$ | Na | |
| 2.85 | $CF_2CHF_2$ | H | H | $CF_3$ | $NO_2$ | Na | |
| 2.86 | $CF_2CHF_2$ | H | H | $CF_3$ | $NO_2$ | Li | |
| 2.87 | $CF_2CHCl_2$ | H | Cl | $CF_3$ | $NO_2$ | K | |
| 2.88 | $CF_2CHCl_2$ | H | H | $CF_3$ | $NO_2$ | K | |
| 2.89 | $CF_2CHCl_2$ | H | H | $CF_3$ | $NO_2$ | Na | |

3. Fortsetzung der Tabelle 2

| Nr. | $R_1$ | $R_2$ | $R_4$ | $R_5$ | $R_6$ | $R_7$ | physik. Konstanten [°C] |
|---|---|---|---|---|---|---|---|
| 2.90 | $CF_2CF_2CF_3$ | H | H | $NO_2$ | $CF_3$ | H | 93-99 |
| 2.91 | $CF_2CHFCF_3$ | H | H | $CF_3$ | $NO_2$ | H | |
| 2.92 | $CF_2CHFCF_3$ | H | H | $NO_2$ | $CF_3$ | H | |
| 2.93 | $CF_2CHFCF_3$ | H | Cl | $CF_3$ | $NO_2$ | H | |
| 2.94 | $CHF(CF_3)_2$ | H | H | $NO_2$ | $CF_3$ | H | |
| 2.95 | $CCl_2CHCl_2$ | H | H | $NO_2$ | $CF_3$ | H | |
| 2.96 | $C_7F_{15}$ | H | H | $NO_2$ | $CF_3$ | H | Sirup |
| 2.97 | $C_7F_{15}$ | H | Cl | $NO_2$ | $CF_3$ | H | |
| 2.98 | $CHF_2$ | H | H | $NO_2$ | $CF_3$ | H | |
| 2.100 | $C_6F_{13}$ | H | H | $NO_2$ | $CF_3$ | H | |
| 2.101 | $C_6F_{13}$ | H | Cl | $NO_2$ | $CF_3$ | H | |
| 2.102 | $C_6F_{13}$ | H | H | $CF_3$ | $NO_2$ | H | |
| 2.103 | $C_8F_{17}$ | H | H | $CF_3$ | $NO_2$ | H | |
| 2.104 | $C_8F_{17}$ | H | Cl | $CF_3$ | $NO_2$ | H | |
| 2.105 | $C_8F_{17}$ | H | H | $NO_2$ | $CF_3$ | H | |
| 2.106 | $C_8F_{17}$ | H | Cl | $NO_2$ | $CF_3$ | H | |
| 2.107 | $CF_2Cl$ | H | H | $NO_2$ | $CF_3$ | H | Smp. 105-107 |
| 2.108 | $CFCl_2$ | H | H | $NO_2$ | $CF_3$ | H | |
| 2.109 | $CF_2CHCl_2$ | H | H | $NO_2$ | $CF_3$ | H | |
| 2.110 | $CF_2CHClF$ | H | H | $NO_2$ | $CF_3$ | H | Smp. 91-93 |
| 2.111 | $CF_2CHF_2$ | H | H | $NO_2$ | $CF_3$ | K | Smp. 271-275 |
| 2.112 | $CFBrCF_3$ | H | H | $NO_2$ | $CF_3$ | H | Smp. 135-137 |
| 2.113 | $CFBrCF_3$ | H | H | $NO_2$ | $CF_3$ | H | |
| 2.114 | $CFBrCF_3$ | H | Cl | $CF_3$ | $NO_2$ | H | |
| 2.115 | $CFBrCF_3$ | H | Cl | $NO_2$ | $CF_3$ | H | |
| 2.116 | $CF_2CF_2Br$ | H | H | $NO_2$ | $CF_3$ | H | |
| 2.117 | $CF_2CF_2Br$ | H | H | $CF_3$ | $NO_2$ | H | |
| 2.118 | $C_6H_5$ | H | Cl | $CF_3$ | $NO_2$ | H | Smp. 181-183 |
| 2.119 | $C_6F_5$ | H | H | $CF_3$ | $NO_2$ | H | |
| 2.120 | $C_6H_5$ | H | H | $NO_2$ | $CF_3$ | H | |
| 2.121 | $C_6F_5$ | H | H | $NO_2$ | $CF_3$ | H | |
| 2.122 | $C_6F_5$ | H | Cl | $CF_3$ | $NO_2$ | H | |

Tabelle 3

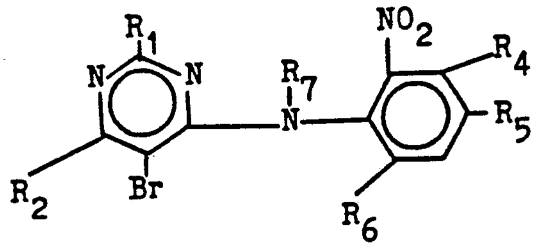

| Nr. | $R_1$ | $R_2$ | $R_4$ | $R_5$ | $R_6$ | $R_7$ | physik. Konstanten [°C] |
|---|---|---|---|---|---|---|---|
| 3.1 | $CH_3$ | H | H | $CF_3$ | $NO_2$ | H | Smp. 88-90 |
| 3.2 | $CHCl_2$ | H | Cl | $CF_3$ | $NO_2$ | H | |
| 3.3 | $CF_3$ | H | H | $CF_3$ | $NO_2$ | K | |
| 3.4 | $CF_2CHF_2$ | H | H | $CF_3$ | $NO_2$ | K | |
| 3.5 | $CCl_2CHCl_2$ | H | Cl | $CF_3$ | $NO_2$ | H | |
| 3.6 | $CCl_2CCl_3$ | H | H | $CF_3$ | $NO_2$ | H | |
| 3.7 | $CCl_2CCl_3$ | H | Cl | $CF_3$ | $NO_2$ | H | |
| 3.8 | $CF_3$ | H | H | $CF_3$ | $NO_2$ | H | |
| 3.9 | $CF_3$ | H | Cl | $CF_3$ | $NO_2$ | H | |
| 3.10 | $CF_2CHF_2$ | H | H | $CF_3$ | $NO_2$ | H | Smp. 154-155 |
| 3.11 | $CF_2CHF_2$ | H | Cl | $CF_3$ | $NO_2$ | H | |
| 3.12 | $CF_2CF_3$ | H | H | $CF_3$ | $NO_2$ | H | |
| 3.13 | $CF_2CF_3$ | H | Cl | $CF_3$ | $NO_2$ | H | |
| 3.14 | $CF_2CHCl_2$ | H | H | $CF_3$ | $NO_2$ | H | |
| 3.15 | $CF_2CHCl_2$ | H | Cl | $CF_3$ | $NO_2$ | H | |
| 3.16 | $CF_2CHFCl$ | H | H | $CF_3$ | $NO_2$ | K | |
| 3.17 | $CF_2CHFCl$ | H | Cl | $CF_3$ | $NO_2$ | H | |
| 3.18 | $CF_2CHFCl$ | H | H | $NO_2$ | $CF_3$ | H | |
| 3.19 | $CF_3$ | H | H | $NO_2$ | $CF_3$ | H | |
| 3.20 | $C\equiv N$ | H | H | $CF_3$ | $NO_2$ | H | |
| 3.21 | $C\equiv N$ | H | Cl | $CF_3$ | $NO_2$ | H | |
| 3.22 | $CHCl_2$ | H | H | $CF_3$ | $NO_2$ | K | |
| 3.23 | $CF_2CHF_2$ | H | H | $CF_3$ | $NO_2$ | Na | |
| 3.24 | $CF_3$ | H | Cl | $CF_3$ | $NO_2$ | K | |
| 3.25 | $CClF_2$ | H | H | $CF_3$ | $NO_2$ | K | |
| 3.26 | $CBrF_2$ | H | H | $CF_3$ | $NO_2$ | H | |
| 3.27 | $CClF_2$ | H | H | $CF_3$ | $NO_2$ | Na | |

1. Fortsetzung der Tabelle 3

| Nr. | $R_1$ | $R_2$ | $R_4$ | $R_5$ | $R_6$ | $R_7$ | physik. Konstanten [°C] |
|---|---|---|---|---|---|---|---|
| 3.28 | $CF_2CHF_2$ | H | H | $NO_2$ | $CF_3$ | H | Smp. 94-95 |
| 3.29 | $CF_2CF_3$ | H | H | $NO_2$ | $CF_3$ | H | |
| 3.30 | $CF_2CF_2CF_3$ | H | H | $CF_3$ | $NO_2$ | H | |
| 3.31 | $CF_2CF_2CF_3$ | H | H | $NO_2$ | $CF_3$ | H | |
| 3.32 | $CF_2CF_2CF_3$ | H | Cl | $NO_2$ | $CF_3$ | H | |
| 3.33 | $CF_2CF_2CF_3$ | H | Cl | $CF_3$ | $NO_2$ | H | |
| 3.34 | $CF_2CHFCF_3$ | H | H | $CF_3$ | $NO_2$ | H | |
| 3.35 | $CF_2CHFCF_3$ | H | Cl | $CF_3$ | $NO_2$ | H | |
| 3.36 | $CF_2CHFCF_3$ | H | H | $NO_2$ | $CF_3$ | H | |
| 3.37 | $(CF_2)_6CF_3$ | H | H | $NO_2$ | $CF_3$ | H | |
| 3.38 | $(CF_2)_6CF_3$ | H | H | $CF_3$ | $NO_2$ | H | |
| 3.39 | $(CF_2)_6CF_3$ | H | Cl | $CF_3$ | $NO_2$ | H | |
| 3.40 | $(CF_2)_6CF_3$ | H | Cl | $NO_2$ | $CF_3$ | H | |
| 3.41 | $CF_2CHCl_2$ | H | H | $NO_2$ | $CF_3$ | H | |
| 3.42 | $CF_2CHCl_2$ | H | Cl | $NO_2$ | $CF_3$ | H | |
| 3.43 | $CClF_2$ | H | H | $NO_2$ | $CF_3$ | H | |
| 3.44 | $CClF_2$ | H | Cl | $NO_2$ | $CF_3$ | H | |
| 3.45 | $(CF_2)_5CF_3$ | H | H | $NO_2$ | $CF_3$ | H | |
| 3.46 | $(CF_2)_5CF_3$ | H | H | $CF_3$ | $NO_2$ | H | |
| 3.47 | $CClF_2$ | H | H | $CF_3$ | $NO_2$ | H | Smp. 171-179 |
| 3.48 | Cl | H | H | $CF_3$ | $NO_2$ | H | Smp. 147-148 |
| 3.49 | $CH_3$ | H | H | $NO_2$ | $CF_3$ | H | Smp. 139-136 |
| 3.50 | $C_6F_5$ | H | H | $NO_2$ | $CF_3$ | H | |
| 3.51 | $C_6F_5$ | H | H | $CF_3$ | $NO_2$ | H | |

Tabelle 4

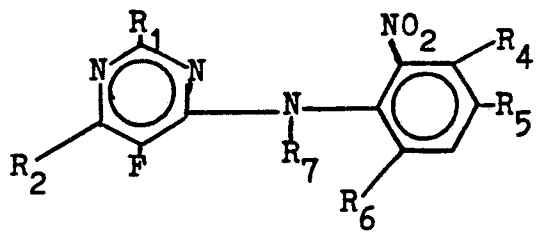

| Nr. | $R_1$ | $R_2$ | $R_4$ | $R_5$ | $R_6$ | $R_7$ | physik. Konstanten [°C] |
|---|---|---|---|---|---|---|---|
| 4.1 | $CF_3$ | $CH_3$ | H | $CF_3$ | $NO_2$ | K | |
| 4.2 | $CF_3$ | H | H | $CF_3$ | $NO_2$ | K | |
| 4.3 | $CF_2CHF_2$ | H | H | $CF_3$ | $NO_2$ | H | |
| 4.4 | $CF_2CHF_2$ | H | Cl | $CF_3$ | $NO_2$ | H | |
| 4.5 | Cl | H | Cl | $CF_3$ | $NO_2$ | H | Smp. 167-168 |
| 4.6 | $OCH_3$ | H | Cl | $CF_3$ | $NO_2$ | H | Smp. 158-159 |
| 4.7 | $OCH_2CF_3$ | H | Cl | $CF_3$ | $NO_2$ | H | Smp. 135-138 |
| 4.8 | C≡N | H | H | $CF_3$ | $NO_2$ | H | |
| 4.9 | C≡N | H | Cl | $CF_3$ | $NO_2$ | H | |

Tabelle 5

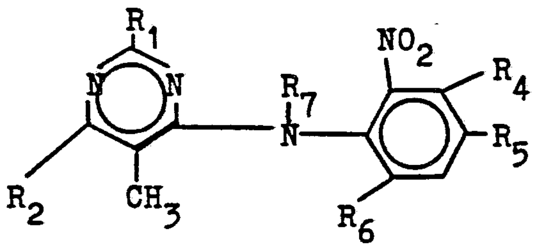

| Nr. | $R_1$ | $R_2$ | $R_4$ | $R_5$ | $R_6$ | $R_7$ | physik. Konstanten [°C] |
|---|---|---|---|---|---|---|---|
| 5.1 | $CH_3$ | H | Cl | $CF_3$ | $NO_2$ | H | |
| 5.2 | 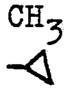 | H | H | $CF_3$ | $NO_2$ | H | |
| 5.3 | $CF_2CHF_2$ | H | H | $CF_3$ | $NO_2$ | K | |
| 5.4 | $CF_3$ | H | Cl | $CF_3$ | $NO_2$ | H | |
| 5.5 | $CCl_2CCl_3$ | H | Cl | $CF_3$ | $NO_2$ | H | |
| 5.6 | $CF_2CHF_2$ | H | Cl | $CF_3$ | $NO_2$ | K | |
| 5.7 | $CF_2CF_3$ | H | Cl | $CF_3$ | $NO_2$ | H | |
| 5.8 | $CF_2CHF_2$ | H | Cl | $CF_3$ | $NO_2$ | H | Smp. 135-137 |
| 5.9 | $CF_3$ | H | H | $CF_3$ | $NO_2$ | H | |
| 5.10 | $CF_2CHF_2$ | H | H | $CF_3$ | $NO_2$ | H | Smp. 139-140 |
| 5.11 | $CF_2CF_3$ | H | H | $CF_3$ | $NO_2$ | H | |
| 5.12 | $CF_2CF_2CF_3$ | H | H | $CF_3$ | $NO_2$ | H | |
| 5.13 | H | H | H | $CF_3$ | $NO_2$ | H | |
| 5.14 | $SCH_3$ | Cl | Cl | $CF_3$ | $NO_2$ | H | Smp. 169-172 |
| 5.15 | $SCH_3$ | Cl | H | $CF_3$ | $NO_2$ | H | Smp. 213-216 |
| 5.16 | $SCH_3$ | Cl | H | $NO_2$ | $CF_3$ | H | Smp. 158-160 |
| 5.17 | $C\equiv N$ | H | H | $CF_3$ | $NO_2$ | H | |
| 5.18 | $C\equiv N$ | H | Cl | $CF_3$ | $NO_2$ | H | |
| 5.19 | Cl | Cl | Cl | $CF_3$ | $NO_2$ | H | Smp. 184-187 |
| 5.20 | Cl | Cl | H | $NO_2$ | $CF_3$ | H | Smp. 207-208 |

Tabelle 6

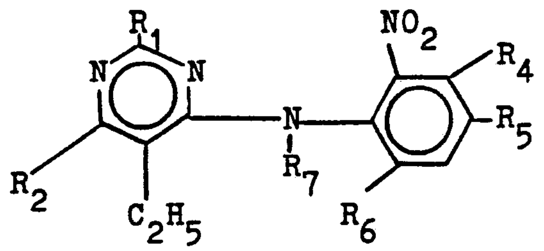

| Nr. | $R_1$ | $R_2$ | $R_4$ | $R_5$ | $R_6$ | $R_7$ | physik. Konstanten [°C] |
|---|---|---|---|---|---|---|---|
| 6.1 | $CF_3$ | $CH_3$ | H | $CF_3$ | $NO_2$ | K | |
| 6.2 | $CF_3$ | H | H | $CF_3$ | $NO_2$ | H | |
| 6.3 | $CF_2CHF_2$ | H | H | $CF_3$ | $NO_2$ | H | |
| 6.4 | $CF_2CHF_2$ | H | Cl | $CF_3$ | $NO_2$ | Na | |
| 6.5 | $CF_2CF_3$ | H | H | $CF_3$ | $NO_2$ | H | |
| 6.6 | $CH_3$ | H | Cl | $CF_3$ | $NO_2$ | H | Smp. 134-136 |
| 6.7 | $CH_3$ | Cl | H | $CF_3$ | $NO_2$ | K | |

Tabelle 7

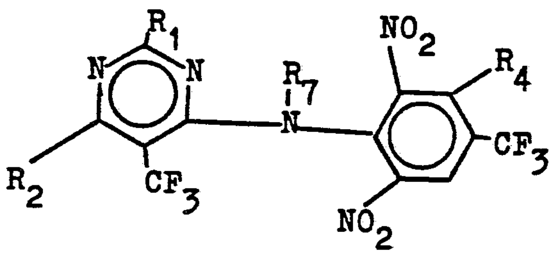

| Nr. | $R_1$ | $R_2$ | $R_4$ | $R_5$ | $R_6$ | $R_7$ | physik. Konstanten [°C] |
|---|---|---|---|---|---|---|---|
| 7.1 | $CCl_2CCl_3$ | H | Cl | $CF_3$ | $NO_2$ | H | |
| 7.2 | H | Cl | Cl | $CF_3$ | $NO_2$ | H | |
| 7.3 | $CH_3$ | H | Cl | $CF_3$ | $NO_2$ | H | |
| 7.4 | $CClF_2$ | H | H | $CF_3$ | $NO_2$ | Na | |
| 7.5 | $CF_2CHF_2$ | H | H | $CF_3$ | $NO_2$ | K | |
| 7.6 | $C_7F_{15}$ | H | H | $CF_3$ | $NO_2$ | H | |
| 7.7 | C≡N | H | H | $CF_3$ | $NO_2$ | H | |
| 7.8 | C≡N | H | Cl | $CF_3$ | $NO_2$ | K | |
| 7.9 | $CH_3$ | $OCH_3$ | H | $CF_3$ | $NO_2$ | H | |
| 7.10 | $CH_3$ | $OCH_3$ | Cl | $CF_3$ | $NO_2$ | H | Smp. 91-95 |
| 7.11 | $CH_3$ | $OCH_3$ | Cl | $CF_3$ | $NO_2$ | K | Smp. 111-115 |
| 7.12 | $CH_3$ | $OCH_3$ | H | $CF_3$ | $NO_2$ | K | Smp. 109-110 |

Tabelle 8

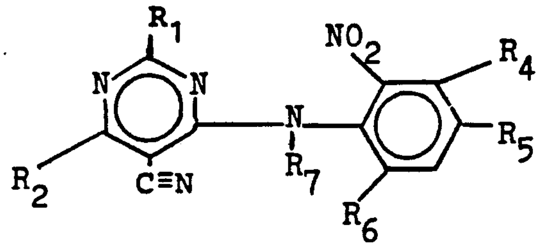

| Nr. | $R_1$ | $R_2$ | $R_4$ | $R_5$ | $R_6$ | $R_7$ | physik. Konstanten [°C] |
|---|---|---|---|---|---|---|---|
| 8.1 | H | H | H | $CF_3$ | $NO_2$ | H | |
| 8.2 | $CF_2CHF_2$ | H | H | $CF_3$ | $NO_2$ | K | Smp. >260 |
| 8.3 | $CF_3$ | H | Cl | $CF_3$ | $NO_2$ | H | |
| 8.4 | $CF_3$ | H | Cl | $CF_3$ | $NO_2$ | H | |
| 8.5 | $CF_3$ | H | H | $CF_3$ | $NO_2$ | H | |
| 8.6 | $CF_2CF_2CF_3$ | H | H | $CF_3$ | $NO_2$ | H | |
| 8.7 | $CF_2CF_2CF_3$ | H | Cl | $CF_3$ | $NO_2$ | H | |
| 8.8 | $CF_2CHFCl$ | H | H | $CF_3$ | $NO_2$ | H | |
| 8.9 | $CF_2CHCl_2$ | H | Cl | $CF_3$ | $NO_2$ | H | |
| 8.10 | $CF_2CHF_2$ | H | H | $CF_3$ | $NO_2$ | H | Smp. 131-132 |
| 8.11 | $CF_2CHF_2$ | H | Cl | $CF_3$ | $NO_2$ | H | Smp. 185-186 |
| 8.12 | 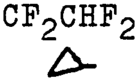 | H | H | $CF_3$ | $NO_2$ | H | |
| 8.13 | 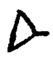 | H | Cl | $CF_3$ | $NO_2$ | H | |
| 8.14 | $CH_3$ | H | H | $CF_3$ | $NO_2$ | H | Smp. 160-162 |
| 8.15 | $CH_3$ | H | Cl | $CF_3$ | $NO_2$ | H | |
| 8.16 | 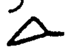 | H | H | $CF_3$ | $NO_2$ | K | |
| 8.17 | 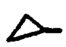 | H | Cl | $CF_3$ | $NO_2$ | K | |
| 8.18 | $CF_3$ | $CF_3$ | H | $CF_3$ | $NO_2$ | H | |
| 8.19 | $CF_3$ | $CF_3$ | Cl | $CF_3$ | $NO_2$ | H | |
| 8.20 | C≡N | H | H | $CF_3$ | $NO_2$ | H | |
| 8.21 | C≡N | H | Cl | $CF_3$ | $NO_2$ | H | |
| 8.22 | $CH_3$ | H | H | $NO_2$ | $CF_3$ | H | |
| 8.23 | $CH_3$ | H | Cl | $NO_2$ | $CF_3$ | H | |
| 8.24 | $CH_3$ | H | H | $CF_3$ | $NO_2$ | K | Smp. 280-281 |
| 8.25 | $CH_3$ | H | Cl | $CF_3$ | $NO_2$ | K | |

1. Fortsetzung der Tabelle 8

| Nr. | $R_1$ | $R_2$ | $R_4$ | $R_5$ | $R_6$ | $R_7$ | physik. Konstanten [°C] |
|---|---|---|---|---|---|---|---|
| 8.26 | $CF_2CHF_2$ | H | H | $NO_2$ | $CF_2$ | H | Smp. 158-159 |
| 8.27 | $CClF_2$ | H | H | $CF_3$ | $NO_2$ | H | Smp. 155-157 |
| 8.28 | $CClF_2$ | H | Cl | $CF_3$ | $NO_2$ | H | Smp. 137-140 |
| 8.29 | $CClF_2$ | H | H | $CF_3$ | $NO_2$ | K | Smp. >260 |

Tabelle 9

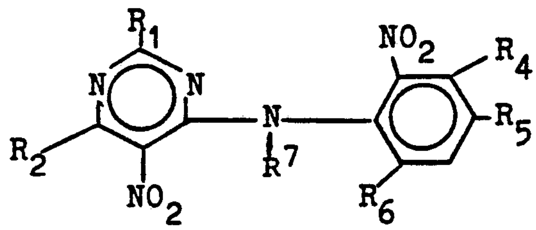

| Nr. | $R_1$ | $R_2$ | $R_4$ | $R_5$ | $R_6$ | $R_7$ | physik. Konstanten [°C] |
|---|---|---|---|---|---|---|---|
| 9.1 | $CH_3$ | H | Cl | $NO_2$ | $CF_3$ | K | |
| 9.2 | C≡N | H | H | $NO_2$ | $CF_3$ | Na | |
| 9.3 | $CF_3$ | H | H | $CF_3$ | $NO_2$ | H | |
| 9.4 | $CF_3$ | H | Cl | $CF_3$ | $NO_2$ | H | |
| 9.5 | $C_2F_5$ | H | H | $CF_3$ | $NO_2$ | K | |
| 9.6 | $C_2F_5$ | H | Cl | $CF_3$ | $NO_2$ | H | |
| 9.7 | $CF_2CHF_2$ | H | H | $CF_3$ | $NO_2$ | H | |
| 9.8 | $CF_2CHF_2$ | H | Cl | $CF_3$ | $NO_2$ | H | |
| 9.9 | $OCH_3$ | H | Cl | $CF_3$ | $NO_2$ | H | Smp. 178-179 |
| 9.10 | $O(CH_2)_2OCH_3$ | H | Cl | $CF_3$ | $NO_2$ | H | Smp. 125-126 |
| 9.11 | $OCH_2CF_3$ | H | Cl | $CF_3$ | $NO_2$ | H | Smp. 138-139 |
| 9.12 | H | $OCH_3$ | Cl | $CF_3$ | $NO_2$ | H | Smp. 184-186 |
| 9.13 | H | $NH_2$ | Cl | $CF_3$ | $NO_2$ | H | Smp. 187-189 |
| 9.14 | H | $SCH_3$ | Cl | $CF_3$ | $NO_2$ | H | Smp. 185 |
| 9.15 | H | $OCH_2CF_3$ | Cl | $CF_3$ | $NO_2$ | H | Smp. 164-167 |
| 9.16 | $SCH_3$ | $OCH_3$ | Cl | $CF_3$ | $NO_2$ | H | Smp. 181-183 |
| 9.17 | $SCH_3$ | $NH_2$ | Cl | $CF_3$ | $NO_2$ | H | Smp. 250 (Zers.) |
| 9.18 | $OCH_3$ | $OCH_3$ | Cl | $CF_3$ | $NO_2$ | H | Smp. 164-166 |

Tabelle 10

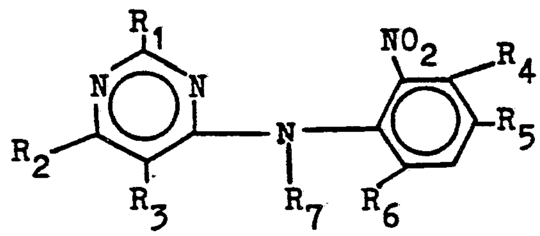

| Nr. | $R_1$ | $R_2$ | $R_3$ | $R_4$ | $R_5$ | $R_6$ | $R_7$ |
|---|---|---|---|---|---|---|---|
| 10.1 | $CF_3$ | H | $CH_2OEt$ | Cl | $CF_3$ | $NO_2$ | H |
| 10.2 | $CF_3$ | H | $CH_2Br$ | H | $CF_3$ | $NO_2$ | H |
| 10.3 | $CF_3$ | H | $CH_2Cl$ | H | $CF_3$ | $NO_2$ | H |
| 10.4 | $CF_3$ | H | $CH_2Br$ | Cl | $CF_3$ | $NO_2$ | H |
| 10.5 | $CF_3$ | H | $Ch_2Cl$ | Cl | $CF_3$ | $NO_2$ | H |
| 10.6 | $CF_3$ | $CH_3$ | $CHCl_2$ | H | $CF_3$ | $NO_2$ | H |
| 10.7 | $CF_2CHF_2$ | H | J | H | $CF_3$ | $NO_2$ | K |
| 10.8 | $CF_2CHF_2$ | H | J | H | $CF_3$ | $NO_2$ | H |
| 10.9 | $CF_2CF_3$ | H | J | H | $CF_3$ | $NO_2$ | Na |
| 10.10 | $CF_2CF_3$ | H | J | Cl | $CF_3$ | $NO_2$ | K |

Beispiel 11.1

N-(3-Chlor-2,6-dinitro-4-trifluormethyl-phenyl)-2-trichlormethyl-4-pyrimidinylamin

Zu einer Lösung von 17,0 g (0,08 mol) 4-Amino-2-trichlormethylpyrimidin in 500 ml wasserfreiem THF wurden bei -5 °C 22,4 g (0,4 mol) KOH-Pulver gefügt. Anschließend tropfte man eine Lösung von 24,4 g (0,08 mol) 2,4-Dichlor-3,5-dinitro-benzotrifluorid in 100 ml abs. THF so hinzu, daß die Temperatur nicht über 0 °stieg. Nach 5-stündiger Nachrührzeit wurden die festen Bestandteile abgesaugt und das Lösungsmittel i. Vak. eingedampft. Der Rückstand wurde in Wasser aufgenommen und mit verdünnter Salzsäure angesäuert. Der ausgefallene Feststoff wurde abgesaugt, getrocknet und aus Diisopropylether umkristillisiert. Smp.: 81-83 °C

Beispiel 11.42

Kalium 5-Cyano-N-(2,6-dinitro-4-trifluormethyl-phenyl)-2-trichlormethyl-4-pyrimidinyl-aminat

Zu einer Lösung von 5,9 g (0,25 mol) 4-Amino-2-trichlormethyl-pyrimidincarbonitril-(5) in 50 ml wasserfreiem THF wurden bei -5 °C 7,0 g (0,125 mol) KOH-Pulver gefügt. Danach tropfte man eine Lösung von 6,8 g (0,025 mol) 4-Chlor-3,5-dinitro-benzotrifluorid in 50 mol abs. THF innerhalb von 30 Minuten so hinzu, daß die Temperatur nicht über 0 °C stieg. Man ließ 4 h nachrühren, saugte die festen Bestandteile ab und

dampfte das Lösungsmittel im Vakuum ab. Der Rückstand wurde in Wasser aufgenommen und mit Chloroform extrahiert. Die vereinigten organischen Phasen wurden über $Na_2SO_4$ getrocknet und im Vakuum eingeengt. Man erhielt einen dunkelroten Feststoff, der aus Ethylacetat umkristallisiert wurde. Smp.: 239-242 °C

Analog Beispiel 11.1 lassen sich die Verbindungen 11.2 bis 11.41 sowie 11.68 bis 11.70 synthetisieren und analog Beipeil 11.41 die Verbindungen 11.43 bis 11.67 sowie 11.71 herstellen (siehe Tabelle 11).

Tabelle 11

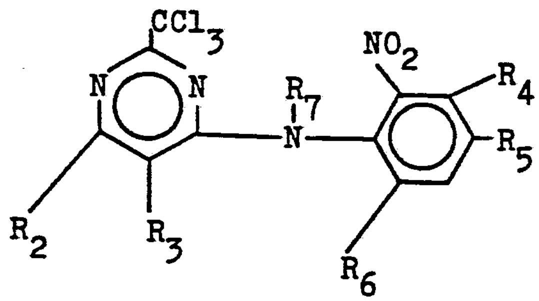

| Nr. | $R_2$ | $R_3$ | $R_4$ | $R_5$ | $R_6$ | $R_7$ | physik. Konstanten [°C] |
|---|---|---|---|---|---|---|---|
| 11.1 | H | H | Cl | $CF_3$ | $NO_2$ | H | Smp. 81-83 |
| 11.2 | H | H | H | $CF_3$ | $NO_2$ | H | |
| 11.3 | $CH_3$ | H | H | $CF_3$ | $NO_2$ | H | Smp. 172-73 |
| 11.4 | $CH_3$ | H | Cl | $CF_3$ | $NO_2$ | H | |
| 11.5 | Cl | H | H | $CF_3$ | $NO_2$ | H | |
| 11.6 | Cl | H | Cl | $CF_3$ | $NO_2$ | H | |
| 11.7 | H | H | H | $NO_2$ | $CF_3$ | H | Smp. 60-62 |
| 11.8 | H | H | Cl | $NO_2$ | $CF_3$ | H | |
| 11.9 | H | Cl | H | $CF_3$ | $NO_2$ | H | Smp. 139-42 |
| 11.10 | H | Cl | Cl | $CF_3$ | $NO_2$ | H | |
| 11.11 | H | Cl | H | $NO_2$ | $CF_3$ | H | Smp. 159-60 |
| 11.12 | $CH_3$ | Cl | H | $CF_3$ | $NO_2$ | H | Smp. 153-55 |
| 11.13 | $CH_3$ | Cl | Cl | $CF_3$ | $NO_2$ | H | Smp. 158-59 |
| 11.14 | $ClCH_2$ | Cl | H | $CF_3$ | $NO_2$ | H | |
| 11.15 | $ClCH_2$ | Cl | Cl | $CF_3$ | $NO_2$ | H | |
| 11.16 | Cl | Cl | H | $CF_3$ | $NO_2$ | H | Smp. 132-35 |
| 11.17 | Cl | Cl | Cl | $CF_3$ | $NO_2$ | H | |
| 11.18 | NHMe | Cl | Cl | $CF_3$ | $NO_2$ | H | |
| 11.19 | NHEt | Cl | Cl | $CF_3$ | $NO_2$ | H | |
| 11.20 | NHMe | Cl | H | $CF_3$ | $NO_2$ | H | |
| 11.21 | NHEt | Cl | H | $CF_3$ | $NO_2$ | H | |
| 11.22 | $NMe_2$ | Cl | H | $CF_3$ | $NO_2$ | H | |
| 11.23 | $NMe_2$ | Cl | Cl | $CF_3$ | $NO_2$ | H | |
| 11.24 | 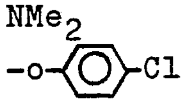 | F | H | $NO_2$ | $CF_3$ | H | |

1. Fortsetzung der Tabelle 11

| Nr. | $R_2$ | $R_3$ | $R_4$ | $R_5$ | $R_6$ | $R_7$ | physik. Konstanten [°C] |
|---|---|---|---|---|---|---|---|
| 11.25 | H | Br | H | $CF_3$ | $NO_2$ | H | |
| 11.26 | H | Br | Cl | $CF_3$ | $NO_2$ | H | |
| 11.27 | Cl | Br | H | $CF_3$ | $NO_2$ | H | Smp. 138-141 |
| 11.28 | Cl | Br | Cl | $CF_3$ | $NO_2$ | H | Smp. 110-112 |
| 11.29 | H | F | H | $CF_3$ | $NO_2$ | H | |
| 11.30 | H | F | Cl | $CF_3$ | $NO_2$ | H | |
| 11.31 | H | $CH_3$ | Cl | $CF_3$ | $NO_2$ | H | |
| 11.32 | H | $CH_3$ | H | $CF_3$ | $NO_2$ | H | |
| 11.33 | H | Et | H | $CF_3$ | $NO_2$ | H | |
| 11.34 | H | Et | Cl | $CF_3$ | $NO_2$ | H | |
| 11.35 | H | $CF_3$ | H | $CF_3$ | $NO_2$ | H | |
| 11.36 | H | C≡N | H | $CF_3$ | $NO_2$ | H | Smp. 144-145 |
| 11.37 | H | C≡N | Cl | $CF_3$ | $NO_2$ | H | |
| 11.38 | H | $NO_2$ | H | $CF_3$ | $NO_2$ | H | |
| 11.39 | H | $NO_2$ | Cl | $CF_3$ | $NO_2$ | H | |
| 11.40 | Cl | C≡N | H | $CF_3$ | $NO_2$ | H | |
| 11.41 | Cl | C≡N | Cl | $CF_3$ | $NO_2$ | H | |
| 11.42 | H | C≡N | H | $CF_3$ | $NO_2$ | K | Smp. 239-242 |
| 11.43 | H | H | H | $CF_3$ | $NO_2$ | K | semikrist. |
| 11.44 | H | H | Cl | $CF_3$ | $NO_2$ | K | Smp. 213-215 |
| 11.45 | $CH_3$ | H | H | $CF_3$ | $NO_2$ | K | Smp. 115-118 |
| 11.46 | $CH_3$ | H | Cl | $CF_3$ | $NO_2$ | K | Smp. 59-60 |
| 11.47 | Cl | H | H | $CF_3$ | $NO_2$ | K | Smp. 203-105 |
| 11.48 | Cl | H | Cl | $CF_3$ | $NO_2$ | K | |
| 11.49 | OMe | $CH_3$ | Cl | $CF_3$ | $NO_2$ | H | |
| 11.50 | H | H | H | $NO_2$ | $CF_3$ | K | |
| 11.51 | H | Cl | H | $CF_3$ | $NO_2$ | K | Smp. 163-166 |
| 11.52 | H | Cl | Cl | $CF_3$ | $NO_2$ | K | Smp. 227-230 |
| 11.53 | $CH_3$ | Cl | H | $CF_3$ | $NO_2$ | K | Smp. 211-213 |
| 11.54 | OEt | Et | Cl | $NO_2$ | $CF_3$ | Li | |
| 11.55 | H | Br | H | $CF_3$ | $NO_2$ | K | Smp. 173-175 |

2. Fortsetzung der Tabelle 11

| Nr. | $R_2$ | $R_3$ | $R_4$ | $R_5$ | $R_6$ | $R_7$ | physik. Konstanten [°C] |
|---|---|---|---|---|---|---|---|
| 11.56 | H | Br | Cl | $CF_3$ | $NO_2$ | K | Smp. 250-253 |
| 11.57 | Cl | Cl | H | $CF_3$ | $NO_2$ | K | |
| 11.58 | Cl | Cl | Cl | $CF_3$ | $NO_2$ | K | |
| 11.59 | H | $CH_3$ | Cl | $CF_3$ | $NO_2$ | K | Smp. 68-72 |
| 11.60 | H | $CH_3$ | H | $CF_3$ | $NO_2$ | K | Smp. 63-66 |
| 11.61 | NHMe | Cl | Cl | $CF_3$ | $NO_2$ | Na | |
| 11.62 | NHEt | Cl | Cl | $CF_3$ | $NO_2$ | Na | |
| 11.63 | $NMe_2$ | Cl | H | $CF_3$ | $NO_2$ | Na | |
| 11.64 | H | Cl | H | $CF_3$ | $NO_2$ | $COCH_3$ | |
| 11.65 | H | Cl | H | $CF_3$ | $NO_2$ | $COCH_2Cl$ | |
| 11.66 | H | Br | H | $CF_3$ | $NO_2$ | CO- -Cl | |
| 11.67 | H | Cl | H | $CF_3$ | $NO_2$ | Na | |
| 11.68 | H | Br | H | $NO_2$ | $CF_3$ | H | Smp. 146-149 |
| 11.69 | Cl | Br | H | $NO_2$ | $CF_3$ | H | Smp. 139-141 |
| 11.70 | Cl | Br | H | $CF_3$ | $NO_2$ | H | Smp. 135-136 |
| 11.71 | $CH_3$ | Cl | Cl | $CF_3$ | $NO_2$ | K | Smp. 107-110 |

Et = Ethyl

## C. Biologische Beispiele

### 1. Insektizidie Wirkung

Beispiel 1

Mit Bohnenspinnmilben (Tetranychus urticae, Vollpolulation) stark befallene Bohnenpflanzen (Phaseolus v.) wurden mit der wäßrigen Verdünnung eines Emulsionskonzentrates, das 1000 ppm des jeweiligen Wirkstoffes enthielt, gespritzt. die Mortalität der Milben wurde nach 7 Tagen kontrolliert. 100 % Abtötung wurde mit den Verbindungen gemäß Beispiel 2.12, 11.53, 2.74, 2.51, 2.111, 5.10, 3.10, 3.47, 11.68, 11.27, 2.90, 1.32, 3.28, 11.11, 2.107, 2.72 und 2.77 erzielt.

Beispiel 2

Mit Obstbaumspinnmilben (Panonychus ulmi) stark befallene Apfelbäumchen (Malus communis) wurden wie Beispiel 1 behandelt.
Die Mortalität der Milben wurden nach 8 Tagen kontrolliert. 100 % Abtötung wurde mit den Verbindungen gemäß Beispiel 2.12, 2.74, 2.18, 2.111, 2.107, 5.10, 3.10, 3.47, 11.68, 11.27, 2.90, 1.32, 2.21, 3.28, 2.72, 2.77 und 11.51 erzielt.

Beispiel 3

Mit Kundebohnenblattlaus (Aphis craccivora) stark besetze Ackerbohnen (Vicia faba) wurden mit wäßrigen Verdünnungen von Spritzpulverkonzentraten mit 1000 ppm Wirkstoffgehalt bis zum Stadium des beginnenden Abtropfens besprüht. Die Mortalität der Blattläuse wurde nach 3 Tagen bestimmt. Eine 100 %ige Abtötung konnte mit den Verbindungen gemäß Beispiel 2.12, 8.14, 8.24, 2.36, 11.71, 2.75, 2.111, 3.48, 2.107, 5.10, 3.10, 3.47, 1.68, 2.90, 1.32, 2.21, 1.196, 3.28, 11.11, 2.72, 2.77 und 11.51 erzielt werden.

Beispiel 4

Mit Weisser Fliege (Traleurodes vaporariorum) stark besetzte Bohnenfplanzen wurden mit wäßrigen Suspensionen von Spritzpulverkonzentraten (1000 ppm Wirkstoffgehalt) bis zum beginnenden Abtropfen gespritzt. Nach Aufstellung der Pflanzen im Gewächshaus erfolgte nach 14 Tagen die mikroskopische Kontrolle mit dem Ergebnis jeweils 100 %iger Mortalität bei den Präparaten mit den Wirkstoffen der Beispiele 11.9, 2.13, 2.12, 2.36, 8.27, 11.71, 11.16, 7.11, 2.75, 2.74, 7.11, 11.13, 11.12, 2.18, 2.42, 2.51, 2.111, 3.48, 2.107, 3.10, 3.47, 11.68, 2.90, 2.21, 1.196, 3.28, 2.71, 2.72, 2.76, 2.77, 11.51 und 11.55 erzielt.

Beispiel 5

Auf die Innenseite des Deckels und des Bodens einer Petrischale werden jeweils 1 ml der zu testenden Formulierung emulgiert in Wasser gleichmäßig aufgetragen und nach dem Antrocknen des Belages jeweils 10 Imagines der Hausfliege (Musca domestica) eingegeben. Nach dem Verschließen der Schale werden diese bei Raumtemperatur aufbewahrt und nach 3 Stunden die Mortalität der Versuchstiere bestimmt. Bei 1000 ppm (bezogen auf den Gehalt an Wirkstoff) zeigten die Präparate 2.12, 3.48, 5.10, 2.1, 11.3, 3.10, 3.47, 11.68, 11.27, 1.32, 5.8, 3.1, 2.21, 1.196, 3.28, 11.11, 2.76, 11.51 und 11.55 eine 100 %ige Wirkung.

Beispiel 6

In eine wie in Beipiel 5 behandelte Petrischale werden je 10 Larven (L4) der Deutschen Schabe (Blatella germanica) gesetzt, die Schalen verschlossen und nach 5 Tagen die Mortalität der Versuchstiere bestimmt.

Bei 1000 ppm (bezogen auf den Gehalt an Wirkstoff) zeigten die Präparate 2.12, 8.27, 8.29, 2.107, 5.10, 3.10, 3.47, 11.68, 2.90, 2.21, 1.196, 3.28, 11.51 und 11.55 eine 100 %ige Wirkung.

Beispiel 7

Auf die Innenseite des Bodens einer Petrischale wurden 1 Lage Filterpapier und darauf eine ca. 3-5 ml große Menge halbsynthetische Futterdiät aufgebracht. Nach dem Erkalten wird die zu testende Formulierung mit Wasser auf die Oberfläche des Futters und des Filterpapiers in fallenden Konzentrationen gesprüht und nach dem Antrocknen des Spritzbelages je 10 Larven (L3-L4) des gemeinen Baumwollwurms (Prodenia litura) eingesetzt.
Die mit Deckel verschlossenen Petrischalen wurden 7 Tage bei Raumtemperatur aufbewahrt und danach die Mortalität der Versuchstiere ermittelt.
Bei 100 ppm (bezogen auf den Gehalt an Wirkstoff) zeigten die Präparate 2.76, 2.77, 11.55 2.13, 2.12, 2.74, 2.18, 3.10, 3.47, 11.68, 2.90, 2.21, 11.11. eine 100 %ige Wirkung.

Beispiel 8

In vitro-Test an tropischen Rinderzecken (Boophilus microplus)

In folgender Versuchsanordnung ließ sich die Wirksamkeit der beanspruchten Verbindungen gegen Zecken nachweisen: Zur Herstellung einer geeigneten Wirkstoffzubereitung wurden die Wirkstoffe 10 %ig (G/V) in einer Mischung, bestehend aus Dimethylformamid (85 g), Nonylphenylpolyglykolether (3 g) und oxethyliertes Rizinusöl (7 g), gelöst und die so erahltenen Emulsionskonzentrate mit Wasser auf die Prüfkonzentrationen verdünnt.

In diese Wirkstoffverdünnungen wurden jeweils zehn vollgesogene Weibchen der tropischen Zecke, Boophilus microplus, für fünf Minuten eingetaucht. Die Zecken wurden anschließend auf Filterpapier getrocknet und dann zum Zwecke der Eiablage mit der Rückseite auf einer Klebefolie befestigt. Die Aufbewahrung der Zecken erfolgte im Wärmeschrank bei 28 °C und einer Luftfeuchtigkeit von 90 %.

Zur Kontrolle wurden Zeckenweibchen lediglich in Wasser eingetaucht.

Zur Bewertung der Wirksamkeit wurde zwei Wochen nach der Behandlung die Hemmung der Eiablage herangezogen. Dabei besagen 100 %, daß keine, 0 % daß alle Zecken Eier abgelegt haben.

In diesem Test bewirkten die Verbindungen 11.51, 11.55 und 2.77 in Wirkstoffkonzentrationen von 0,1 % (G/V) jeweils eine 100 % Hemmung der Eiablage.

Beispiel 9

Insektizide Wirkung von Kombinationen mit Endosulfan

Zum Nachweis eines synergistischen Effektes der zu untersuchenden Verbindungen wurden 3 Serien von Versuchen parallel durchgeführt. In je einer Serie wurden die beiden Kombinationspartner alleine in fallenden Konzentrationen in mit künstlicher Futterdiät ausgegossenen Petrischalen entsprechend 600 l/ha gesprüht und nach dem Antrocknen des Spitzbelages je 10 Larven (L3) von Spodoptera littoralis pro Schale eingesetzt. In einer dritten Serie wurden die Schalen zuerst mit dem einen Kombinationspartner in oben geschilderter Weise behandelt und nach dem Abtrocknen die Schalen mit dem zweiten Kombinationspartner zusätzlich in gleicher Weise behandelt. Nach dem Abtrocknen des zweiten Belages wurden wie in der ersten und zweiten Serie je Schale 10 Larven (L3) der genannten Tierart eingesetzt.

Es zeigte sich (Tabelle I), daß die Wirkung der kombinierten Behandlung, bezogen auf die jeweils angewandte Dosierung, weit mehr als die additive Wirkung der Einzelpartner beträgt.

Tabelle I:

| Bsp.Nr. Vbg I 11.11 (A) | Komb.partner Endosulfan (B) | Wirkung der Einzelkomponente | | Wirkung der Kombination |
|---|---|---|---|---|
| (Konz. in ppm) | | (% Mortalität der Larven) | | |
| | | A | B | A+B |
| 63 | 500 | 50 | 0 | 100 |
| 31 | 250 | 0 | 0 | 90 |
| 16 | 125 | 0 | 0 | 0 |

Beispiel 10

Eine staubförmige Formulierung wurde mit Erde gemischt, die mit Meloidogyne incognita verseucht war. Anschließend erfolgte das Abfüllen in Töpfe und die Bepflanzung dieser mit Tomaten. Nach einer Standzeit von 4 Wochen im Gewächshaus wurden die Wertzahlen nach folgendem Schema ermittelt:

| Gallen/Pflanze | Wertzahl |
|---|---|
| 0 | 1 |
| 1-2 | 2 |
| 3-5 | 3 |
| 6-10 | 4 |
| über 150 | 9 |

Mit einer Aufwandmenge von 15 ppm der Wirkstoffe aus Beispiel 8.2, 2.51, 2.111 und 2.107 wurde Wertzahl 1 (= 100 % Wirkung) erzielt.

2. Fungizidie Wirkung

Beispiel 11

Biomalzagar wurde mit den beanspruchten Verbindungen (Tabelle II und III) in Konzentrationen von jeweils 2000 und 500 ppm Wirkstoff versetzt. Nach dem Erstarren des Agars erfolgte die Beimpfung mit verschiedenen Botrytis cinerea-Kulturen, die Benzimidazol-und Iprodion-sensibel bzw. -resistent sind. Jeweils 20 µl einer Sporensuspension wurden auf das Zentrum der Agarplatte aufgebracht. Die Versuche wurden 6 Tage nach Beimpfung ausgewertet. Der Wirkungsgrad der Wirkstoffe wird ausgedrückt in % im Vergleich zur Kontrolle (Agarmedium ohne Wirkstoff).

Tabelle II

Botrytis cinerea, BCM- und Iprodion-sensibler Stamm

| Verbindung gemäß Beispiel Nr. | Wirkungsgrad in % bei ppm Wirkstoff 2000 | 500 |
|---|---|---|
| 11.16 | 100 | 100 |
| 11.42 | 100 | 80 |
| 11.51 | 100 | 100 |
| 11.55 | 100 | 100 |
| 11.60 | 100 | 50 |
| 11.45 | 100 | 50 |
| 11.44 | 100 | 100 |
| 11.52 | 100 | 100 |
| 11.56 | 100 | 80 |
| 11.59 | 100 | 100 |
| 11.46 | 100 | 100 |
| 11.71 | 100 | 50 |
| Kontrolle | 0 | |

Tabelle III

Botrytis cinerea, BCM- und Iprodion-resistenter Stamm

| Verbindung gemäß Beispiel Nr. | Wirkungsgrad in % bei ppm Wirkstoff 2000 | 500 |
|---|---|---|
| 11.16 | 100 | 100 |
| 11.42 | 100 | 50 |
| 11.51 | 100 | 100 |
| 11.55 | 100 | 80 |
| 11.60 | 100 | 50 |
| 11.45 | 100 | 50 |
| 11.44 | 100 | 100 |
| 11.52 | 100 | 100 |
| 11.56 | 100 | 100 |
| 11.59 | 100 | 100 |
| 11.46 | 100 | 100 |
| 11.71 | 100 | 50 |
| Kontrolle | 0 | |

Beispiel 12

Biomalzagar wird mit den beanspruchten Verbindungen (Tabelle IVa und IVb) in Konzentrationen von jeweils 125, 30 und 8 ppm Wirkstoff versetzt. Nach dem Erstarren des Agars erfolgt die Beimpfung mit Pseudocercosporella-Kulturen (BCM-sensibel und BCM-resistent). Jeweils 20 µl einer Sporensuspension werden auf das Zentrum der Agarplatte aufgebracht. Die Versuche werden 6 Tage nach Beimpfung ausgewertet. Der Wirkungsgrad der Wirkstoffe wird ausgedrückt in % im Vergleich zur Kontrolle (Agarmedium ohne Wirkstoff).

Tabelle IVa

| Verbindung gemäß Beispiel Nr. | Wirkungsgrad gegenüber Pseudocercosporella herpotrichoides in % bei ppm Wirkstoff BCM-sensibler Stamm | | |
|---|---|---|---|
| | 125 | 30 | 8 |
| 11.42 | 100 | 100 | 100 |
| 11.51 | 100 | 100 | 100 |
| 11.55 | 100 | 100 | 100 |
| 11.45 | 100 | 80 | - |
| 11.44 | 100 | 100 | 100 |
| 11.56 | 100 | 100 | 100 |
| 11.59 | 100 | 100 | 80 |
| 11.46 | 100 | 100 | 80 |
| Kontrolle | | 0 | |

Tabelle IVb

| Verbindung gemäß Beispiel Nr. | Wirkungsgrad gegenüber Pseudocercosporella herpotrichoides in % bei ppm Wirkstoff | | | | | |
|---|---|---|---|---|---|---|
| | BCM-sensibler Stamm | | | BCM-resistenter Stamm | | |
| | 125 | 30 | 8 | 125 | 30 | 8 |
| 11.68 | 100 | 100 | 100 | 100 | 100 | 100 |
| 11.27 | 100 | 100 | 100 | 100 | 100 | 100 |
| 11.1 | 100 | 100 | 100 | - | - | - |
| 11.71 | 100 | 100 | 100 | 100 | 100 | 100 |
| 11.16 | 100 | 100 | 100 | 100 | 100 | 100 |
| 11.47 | 100 | 100 | 100 | 100 | 100 | 100 |

Beispiel 13

Weinpflanzen, die aus Stecklingen der Plasmopara-anfälligen Sorte Müller-Thurgau gezogen waren, wurden im 4-Blattstadium mit wäßrigen Suspensionen der beanspruchten Verbindungen tropfnaß behandelt. Die Anwendungskonzentrationen betrugen 500, 250 und 125 mg Wirkstoff pro Liter Spritzbrühe.

Nach dem Antrocknen des Spritzbelages wurden die Pflanzen mit einer Zoosporangiensuspension von Plasmopara viticola inokuliert und tropfnaß in eine Klimakammer bei einer Temperatur von ca. 20 °C und einer relativen Luftfeuchtigkeit von ca. 100 % gestellt. Nach 24 Stunden wurden die infizierten Pflanzen der Klimakammer entnommen und in ein Gewächshaus mit einer Temperatur von ca. 23 °C und einer Luftfeuchtigkeit von ca. 80-90 % gebracht.

Nach einer Inkubationszeit von 7 Tagen wurden die Pflanzen über Nacht in die Klimakammer gestellt und die Krankheit zum Ausbruch gebracht. Anschließend erfolgt die Befallsauswertung. Der Befallsgrad wurde in % befallener Blattfläche im Vergleich zu den unbehandelten, infizierten Kontrollpflanzen ausgedrückt und ist in Tabelle V wiedergegeben.

Tabelle V

| Verbindung gemäß Beispiel Nr. | % Plasmopora viticola - Befall bei ppm Wirkstoff | | |
|---|---|---|---|
| | 500 | 250 | 125 |
| 11.42 | 0 | 0 | 0-3 |
| 11.51 | 0 | 0 | 0 |
| 11.55 | 0 | 0 | 0 |
| 11.44 | 0 | 0 | 0-3 |
| 11.52 | 0 | 0 | 0 |
| 11.59 | 0 | 0-3 | 3 |
| 11.46 | 0 | 0 | 0 |
| 11.47 | 0 | 0 | 0-3 |
| 11.16 | 0 | 0 | 0 |
| 11.1 | 0 | 0 | 0 |
| 11.68 | 0 | 0 | 0 |
| unbehandelte, infizierte Pflanzen | | 100 | |

Beispiel 14

Apfelunterlagen (EM IX) wurden im 4-Blattstadium mit den beanspruchten Verbindungen in den Anwendungskonzentrationen von 500, 250 und 125 mg Wirkstoff/Liter Spritzbrühe gleichmäßig behandelt.

Nach Antrocknen des Wirkstoffbelages wurden Pflanzen mit Konidien des Apfelschorfs (Venturia inaequalis) stark inokuliert und tropfnaß in eine Klimakammer gestellt, deren Temperatur ca. 22 °C und deren relative Luftfeuchtigkeit ca. 100 % betrugt. Nach einer Infektionszeit von 48 Stunden kamen die Pflanzen in ein Gewächshaus mit ca. 18 °C und einer relativen Luftfeuchtigkeit von 95-100 %.

Nach einer Inkubationszeit von 14 Tagen wurden die Pflanzen auf Befall mit Apfelschorf (Venturia inaequalis) untersucht. Die Beurteilung des Befalls erfolgte wie üblich nach Augenschein. Der Befallsgrad der Pflanzen mit Apfelschorf wurde in % befallener Blattfläche, bezogen auf unbehandelte, infizierte Pflanzen, ausgedrückt und ist in Tabelle VI wiedergegeben.

Tabelle VI

| Verbindung gemäß Beispiel Nr. | % Schorfbefall bei ppm Wirkstoff 500 | 250 | 125 |
|---|---|---|---|
| 11.51 | 0 | 0 | 0 |
| 11.55 | 0 | 0 | 0 |
| 11.60 | 0 | 0 | 0 |
| 11.45 | 0 | 0 | 0 |
| 11.56 | 0 | 0 | 0-3 |
| 11.46 | 0 | 0 | 0-3 |
| unbehandelte, infizierte Pflanzen | | 100 | |

Beispiel 15

Fungizide synergistische Wirkung von Kombinationen

Kreuzstreifen-Test (Methode in J. Gen. Microbiol 126 (1981), Seite 1-7) in Petrischalen.

Filterpapierstreifen (10 mm breit und 90 mm lang) wurden mit den formulierten Wirkstoffen der Formel I und dem Kombinationspartner (Iprodion) in verschiedenen Konzentrationen gleichmäßig benetzt (ca. 200 µl/Streifen) und auf ein je nach Pilzart unterschiedliches Agar-Medium aufgelegt. Dem Agar wurden zuvor in noch flüssigen Zustand zu Petrischale 0,5 ml Suspensionskultur des Testorganismus (ca. $10^5$-$10^6$ Konidien/ml) zugegeben. Jede Petrischale enthielt einen Filterpapierstreifen mit je einem Vertreter der Formel I und senkrecht dazu einem zweiten Streifen mit dem Kombinationspartner. Die Wirkstoffkonzentrationen wurden durch entsprechende Vorversuche mit den Einzelkomponenten so gewählt, daß die gegenüber dem betreffenden Testorganismus der sogenannten minimalen Hemmstoffkonzentration entsprachen.

Nach ca. 3-4-tägiger Inkubation der Petrischalen bei 22-25°C wurden die Inhibitionszonen im Bereich der sich kreuzenden Teststreifen diametral ausgemessen und die Hemmzone als Maß für den Synergismus bewertet.

Tabelle VII

Kreuzstreifen-Test
Testobjekt: Botrytis cinerea

| Wirkstoff bzw. Kombinationen | | Konz. Wirkstoff ppm | Hemmzonen in mm |
|---|---|---|---|
| Bsp. 11.51 | a) | 500 | 0 |
| | b) | 250 | 0 |
| Iprodion | c) | 500 | 8 |
| | d) | 250 | 0 |
| Wirkstoffkombinationen | a+c | 500+500 | 18 |
| | a+d | 500+250 | 8 |
| | b+c | 250+500 | 12 |
| | b+d | 250+250 | 8 |

**Ansprüche**

1. Schädlingsbekämpfungsmittel, dadurch gekennzeichnet, daß sie eine Verbindung der Formel (I)

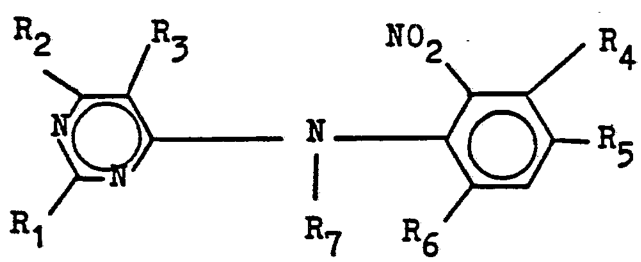

(I)

worin

$R_1$ und $R_2$ unabhängig voneinander Wasserstoff; Halogen; Cyano; ($C_1$-$C_8$)-Alkyl; ($C_1$-$C_8$)-Haloalkyl; ($C_1$-$C_8$)-Alkyl, das ein-oder zweifach durch Nitro, Cyano, ($C_1$-$C_4$)-Alkoxy, ($C_1$-$C_4$)-Alkylthio oder -N($R_8$)($R_9$) substituiert ist; ($C_3$-$C_8$)-Cycloalkyl, das durch ($C_1$-$C_4$)-Alkyl substituiert sein kann; ($C_2$-$C_4$)-Alkenyl; ($C_2$-$C_4$)-Haloalkenyl; ($C_5$-$C_6$)-Cycloalkenyl; eine Gruppe N($R_8$)($R_9$); ($C_1$-$C_8$)-Alkylthio; ($C_1$-$C_8$)-Alkoxy; ($C_1$-$C_8$)-Haloalkoxy; ($C_1$-$C_8$)-Alkylsulfonyl; Phenyl, das gegebenenfalls durch Halogen, Nitro, Cyano, ($C_1$-$C_4$)-Alkyl, ($C_1$-$C_4$)-Alkoxy, ($C_1$-$C_4$)-Alkylthio oder ($C_1$-$C_4$)-Haloalkyl substituiert sein kann oder Phenoxy, das ein-bis dreifach durch Halogen, $NO_2$, ($C_1$-$C_4$)-Alkyl, ($C_1$-$C_4$)-Haloalkyl oder ($C_1$-$C_4$)-Alkoxy substituiert sein kann;

$R_3$ Wasserstoff, Halogen; ($C_1$-$C_8$)-Alkyl; ($C_1$-$C_8$)-Haloalkyl; ($C_2$-$C_4$)-Alkenyl; ($C_2$-$C_4$)-Haloalkenyl; ($C_1$-$C_4$)-Alkoxy; ($C_1$-$C_4$)-Alkythio; Cyano; Nitro; Phenyl, das ein-bis dreifach durch Halogen, Nitro, Cyano; ($C_1$-$C_4$)-Alkyl, ($C_1$-$C_4$)-Alkoxy, ($C_1$-$C_4$)-Alkylthio oder ($C_1$-$C_4$)-Haloalkyl substituiert sein kann; oder Phenoxy, das ein-bis dreifach durch Halogen, $NO_2$, ($C_1$-$C_4$)-Alkyl, ($C_1$-$C_4$)-Haloalkyl oder ($C_1$-$C_4$)-Alkoxy substituiert sein kann,

$R_4$ Wasserstoff, Halogen, ($C_1$-$C_4$)-Alkoxy, ($C_1$-$C_4$)-Alkylthio, eine Gruppe N($R_8$)($R_9$) oder Phenoxy, das ein-bis dreifach durch Halogen, Nitro, ($C_1$-$C_4$)-Alkyl, ($C_1$-$C_4$)-Haloalkyl oder ($C_1$-$C_4$)-Alkoxy substituiert sein kann;

$R_5$ und $R_6$ unabhängig voneinander Nitro oder $CF_3$;

$R_7$ Wasserstoff, eine Gruppe -$COR_{10}$ oder ein Kationäquivalent,

$R_8$ und $R_9$ unabhängig voneinander Wasserstoff, $(C_1\text{-}C_4)$-Alkyl oder $(C_3\text{-}C_7)$-Cycloalkyl und
$R_{10}$ Wasserstoff oder $(C_1\text{-}C_4)$-Alkyl bedeuten, enthalten.

2. Mittel gemäß Anspruch 1, dadurch gekennzeichnet, daß in Formel I $R_1$, $R_2$ = $(C_1\text{-}C_8)$-Alkyl, $(C_1\text{-}C_8)$-Haloalkyl; $(C_1\text{-}C_8)$-Alkyl, das ein-oder zweifach durch Nitro, Cyano, $(C_1\text{-}C_4)$-Alkoxy, $(C_1\text{-}C_4)$-Alkylthio oder -N-$(R_8)(R_9)$ substituiert ist, $(C_3\text{-}C_8)$-Cycloalkyl, das durch $(C_1\text{-}C_4)$-Alkyl substituiert sein kann; $(C_2\text{-}C_4)$-Alkenyl; $(C_2\text{-}C_4)$-Haloalkenyl; Phenyl, das gegebenenfalls durch Halogen, Nitro, Cyano, $(C_1\text{-}C_4)$-Alkyl, $(C_1\text{-}C_4)$-Alkoxy, $(C_1\text{-}C_4)$-Alkylthio oder $(C_1\text{-}C_4)$-Haloalkyl substituiert sein kann oder,
$R_3$ Wasserstoff, Halogen; $(C_1\text{-}C_8)$-Alkyl; $(C_1\text{-}C_8)$-Haloalkyl; Cyano oder Phenoxy, das ein-bis dreifach durch Halogen, $NO_2$, $(C_1\text{-}C_4)$-Alkyl, $(C_1\text{-}C_4)$-Haloalkyl oder $(C_1\text{-}C_4)$-Alkoxy substituiert sein kann;
$R_4$ Wasserstoff; Halogen, $(C_1\text{-}C_4)$-Alkoxy oder $(C_1\text{-}C_4)$-Alkylthio;
$R_5$ und $R_6$ unabhängig voneinander Nitro oder $CF_3$;
$R_7$ Wasserstoff, eine Gruppe $-COR_{10}$ oder ein Kationäquivalent;
$R_8$ und $R_9$ unabhängig von einander Wasserstoff; $(C_1\text{-}C_4)$-Alkyl oder $(C_3\text{-}C_7)$-Cycloalkyl und
$R_{10}$ Wasserstoff oder $(C_1\text{-}C_4)$-Alkyl bedeuten.

3. Mittel gemäß Anspruch 1, dadurch gekennzeichnet, daß in Formel I, $R_1$ $(C_1\text{-}C_8)$-Haloalkyl, $R_2$ Wasserstoff, $R_3$ Halogen, $R_4$ Wasserstoff, $R_5$ und $R_6$ unabhängig von einander $NO_2$ oder $CF_3$ und $R_7$ Wasserstoff bedeuten.

4. Verfahren zur Bekämpfung von tierischen Schädlingen insbesondere Schadinsekten dadurch gekennzeichnet, daß man auf diese oder die von Ihnen befallenen Pflanzen oder Anbauflächen eine wirksame Menge eines Mittels gemäß Ansprüchen 1, 2 oder 3 appliziert.

5. Verwendung von Verbindung der Formel I von Ansprüchen 1, 2 oder 3 zur Bekämpfung von tierischen Schädlingen.

6. Verbindungen der Formel I, worin $R_1$ $CCl_3$ bedeutet und die übrigen Reste die Definitionen wie in Anspruch 1, 2 oder 3 besitzen.

7. Verbindungen der Formel I, von Anspruch 6, worin $R_1$ = $CCl_3$, $R_2$ = Halogen, $(C_1\text{-}C_8)$-Alkyl, $(C_1\text{-}C_8)$-Haloalkyl; $R_3$ = H, Halogen oder Cyano; $R_4$ H = oder Halogen; $R_5$, $R_6$ = Nitro oder $CF_3$ und $R_7$ = H bedeuten.

8. Verfahren zur Herstellung von Verbindungen der Formel I von Anspruch 6 oder 7, dadurch gekennzeichnet, daß man eine Verbindung der Formel II mit einer Verbindung der Formel III

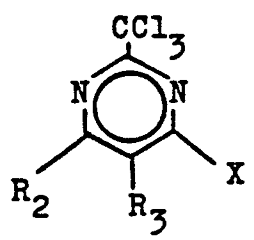
(II)

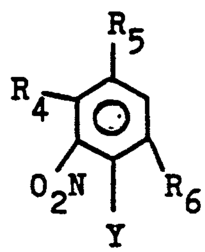
(III)

wobei X, Y = Halogen oder $NH_2$ bedeuten, wobei im Falle X = Halogen Y = $NH_2$ und im Falle X = $NH_2$ Y = Halogen sein muß, in Gegenwart einer Base umsetzt.

9. Verfahren zur Bekämpfung von Schadpilzen, dadurch gekennzeichnet, daß man auf diese oder die von Ihnen befallenen Pflanzen oder Anbauflächen eine wirksame Menge einer Verbindung von Anspruch 6 oder 7 appliziert.

Patentansprüche für die folgenden Vertragsstaaten: Österreich und Spanien.

1. Schädlingsbekämpfungsmittel, dadurch gekennzeichnet, daß sie eine Verbindung der Formel (I)

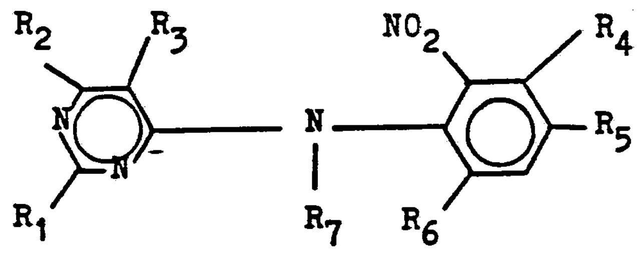

(I)

worin

$R_1$ und $R_2$ unabhängig voneinander Wasserstoff; Halogen; Cyano; ($C_1$-$C_8$)-Alkyl; ($C_1$-$C_8$)-Haloalkyl; ($C_1$-$C_8$)-Alkyl, das ein-oder zweifach durch Nitro, Cyano, ($C_1$-$C_4$)-Alkoxy, ($C_1$-$C_4$)-Alkylthio oder -N($R_8$)($R_9$) substituiert ist; ($C_3$-$C_8$)-Cycloalkyl, das durch ($C_1$-$C_4$)-Alkyl substituiert sein kann; ($C_2$-$C_4$)-Alkenyl; ($C_2$-$C_4$)-Haloalkenyl; ($C_5$-$C_6$)-Cycloalkenyl; eine Gruppe N($R_8$)($R_9$); ($C_1$-$C_8$)-Alkylthio; ($C_1$-$C_8$)-Alkoxy; ($C_1$-$C_8$)-Haloalkoxy; ($C_1$-$C_8$)-Alkylsulfonyl; Phenyl, das gegebenenfalls durch Halogen, Nitro, Cyano, ($C_1$-$C_4$)-Alkyl, ($C_1$-$C_4$)-Alkoxy, ($C_1$-$C_4$)-Alkylthio oder ($C_1$-$C_4$)-Haloalkyl substituiert sein kann oder Phenoxy, das ein-bis drei fach durch Halogen, $NO_2$, ($C_1$-$C_4$)-Alkyl, ($C_1$-$C_4$)-Haloalkyl oder ($C_1$-$C_4$)-Alkoxy substituiert sein kann;

$R_3$ Wasserstoff, Halogen; ($C_1$-$C_8$)-Alkyl; ($C_1$-$C_8$)-Haloalkyl; ($C_2$-$C_4$)-Alkenyl; ($C_2$-$C_4$)-Haloalkenyl; ($C_1$-$C_4$)-Alkoxy; ($C_1$-$C_4$)-Alkylthio; Cyano; Nitro; Phenyl, das ein-bis dreifach durch Halogen, Nitro, Cyano; ($C_1$-$C_4$)-Alkyl, ($C_1$-$C_4$)-Alkoxy, ($C_1$-$C_4$)-Alkylthio oder ($C_1$-$C_4$)-Haloalkyl substituiert sein kann; oder Phenoxy, das ein-bis dreifach durch Halogen, $NO_2$, ($C_1$-$C_4$)-Alkyl, ($C_1$-$C_4$)-Haloalkyl oder ($C_1$-$C_4$)-Alkoxy substituiert sein kann,

$R_4$ Wasserstoff, Halogen, ($C_1$-$C_4$)-Alkoxy, ($C_1$-$C_4$)-Alkylthio, eine Gruppe N($R_8$)($R_9$) oder Phenoxy, das ein-bis dreifach durch Halogen, Nitro, ($C_1$-$C_4$)-Alkyl, ($C_1$-$C_4$)-Haloalkyl oder ($C_1$-$C_4$)-Alkoxy substituiert sein kann;

$R_5$ und $R_6$ unabhängig voneinander Nitro oder $CF_3$;

$R_7$ Wasserstoff, eine Gruppe -$COR_{10}$ oder ein Kationäquivalent,

$R_8$ und $R_9$ unabhängig voneinander Wasserstoff, ($C_1$-$C_4$)-Alkyl oder ($C_3$-$C_7$)-Cycloalkyl und

$R_{10}$ Wasserstoff oder ($C_1$-$C_4$)-Alkyl bedeuten, enthalten.

2. Mittel gemäß Anspruch 1, dadurch gekennzeichnet, daß in Formel I $R_1$, $R_2$ = ($C_1$-$C_8$)-Alkyl, ($C_1$-$C_8$)-Haloalkyl; ($C_1$-$C_8$)-Alkyl, das ein-oder zweifach durch Nitro, Cyano, ($C_1$-$C_4$)-Alkoxy, ($C_1$-$C_4$)-Alkylthio oder -N-($R_8$)($R_9$) substituiert ist, ($C_3$-$C_8$)-Cycloalkyl, das durch ($C_1$-$C_4$)-Alkyl substituiert sein kann; ($C_2$-$C_4$)-Alkenyl; ($C_2$-$C_4$)-Haloalkenyl; Phenyl, das gegebenenfalls durch Halogen, Nitro, Cyano, ($C_1$-$C_4$)-Alkyl, ($C_1$-$C_4$)-Alkoxy, ($C_1$-$C_4$)-Alkylthio oder ($C_1$-$C_4$)-Haloalkyl substituiert sein kann oder,

$R_3$ Wasserstoff, Halogen; ($C_1$-$C_8$)-Alkyl; ($C_1$-$C_8$)-Haloalkyl; Cyano oder Phenoxy, das ein-bis dreifach durch Halogen, $NO_2$, ($C_1$-$C_4$)-Alkyl, ($C_1$-$C_4$)-Haloalkyl oder ($C_1$-$C_4$)-Alkoxy substituiert sein kann;

$R_4$ Wasserstoff; Halogen, ($C_1$-$C_4$)-Alkoxy oder ($C_1$-$C_4$)-Alkylthio;

$R_5$ und $R_6$ unabhängig voneinander Nitro oder $CF_3$;

$R_7$ Wasserstoff, eine Gruppe -$COR_{10}$ oder ein Kationäquivalent;

$R_8$ und $R_9$ unabhängig von einander Wasserstoff; ($C_1$-$C_4$)-Alkyl oder ($C_3$-$C_7$)-Cycloalkyl und

$R_{10}$ Wasserstoff oder ($C_1$-$C_4$)-Alkyl bedeuten.

3. Mittel gemäß Anspruch 1, dadurch gekennzeichnet, daß in Formel I, $R_1$ ($C_1$-$C_8$)-Haloalkyl, $R_2$ Wasserstoff, $R_3$ Halogen, $R_4$ Wasserstoff, $R_5$ und $R_6$ unabhängig von einander $NO_2$ oder $CF_3$ und $R_7$ Wasserstoff bedeuten.

4. Verfahren zur Bekämpfung von tierischen Schädlingen insbesondere Schadinsekten dadurch gekennzeichnet, daß man auf diese oder die von Ihnen befallenen Pflanzen oder Anbauflächen eine wirksame Menge eines Mittels gemäß Ansprüchen 1, 2 oder 3 appliziert.

5. Verwendung von Verbindung der Formel I von Ansprüchen 1, 2 oder 3 zur Bekämpfung von tierischen Schädlingen.

6. Verfahren zur Herstellung von Verbindungen der Formel I von Anspruch 1, 2 oder 3 worin $R_1$ = $CCl_3$ bedeutet, dadurch gekennzeichnet, daß man eine Verbindung der Formel II mit einer Verbindung der Formel III

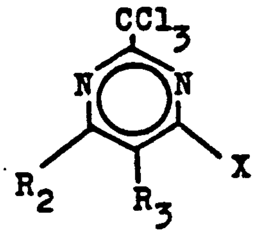

(II)

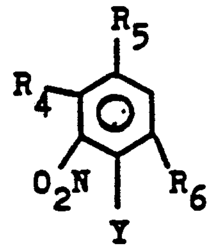

(III)

wobei X, Y = Halogen oder $NH_2$ bedeuten, wobei im Falle X = Halogen Y = $NH_2$ und im Falle X = $NH_2$ Y = Halogen sein muß in Gegenwart einer Base umsetzt.

7. Verfahren gemäß Anspruch 6, dadurch gekennzeichnet, daß $R_1$ = $CCl_3$, $R_2$ Halogen, ($C_1$-$C_8$)-Alkyl, ($C_1$-$C_8$)-Haloalkyl; $R_3$ = H, Halogen oder Cyano; $R_4$ = H oder Halogen; $R_5$, $R_6$ = Nitro oder $CF_3$ und $R_7$ = H bedeuten.

8. Verfahren zur Bekämpfung von Schadpilzen, dadurch gekennzeichnet, daß man auf diese oder die von Ihnen befallenen Pflanzen oder Anbauflächen eine wirksame Menge einer Verbindung von Anspruch 6 oder 7 appliziert.

Europäisches Patentamt

# EUROPÄISCHER RECHERCHENBERICHT

Nummer der Anmeldung

EP 87107729.3

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | EP - A1 - 0 126 254 (CIBA-GEIGY AG)<br>* Ansprüche 1,12,13,18 *<br>-- | 1,8 | A 01 N 43/54<br>C 07 D 239/42 |
| D,X | EP - A1 - 0 139 613 (CIBA-GEIGY AG)<br>* Ansprüche 1,10,12 *<br>-- | 1,8 | |
| A | EP - A1 - 0 067 630 (SANKKO COMPANY LIMITED)<br>* Zusammenfassung *<br>---- | 8 | |

RECHERCHIERTE SACHGEBIETE (Int. Cl 4)

A 01 N
C 07 D 239/00

Der vorliegende Recherchenbericht wurde für alle Patentansprüche erstellt.

| Recherchenort | Abschlußdatum der Recherche | Prüfer |
|---|---|---|
| WIEN | 02-09-1987 | SCHNASS |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPA Form 1503 03 82